(19) Europäisches Patentamt
European Patent Office
Office européen des brevets

(11) EP 4 174 451 A1

(12) **EUROPEAN PATENT APPLICATION**
published in accordance with Art. 153(4) EPC

(43) Date of publication:
03.05.2023 Bulletin 2023/18

(21) Application number: 21833980.2

(22) Date of filing: 28.06.2021

(51) International Patent Classification (IPC):
$G01F\ 1/66^{(2022.01)}$ $A61B\ 5/026^{(2006.01)}$

(52) Cooperative Patent Classification (CPC):
A61B 5/026; G01F 1/66

(86) International application number:
PCT/JP2021/024345

(87) International publication number:
WO 2022/004650 (06.01.2022 Gazette 2022/01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.06.2020 JP 2020113104

(71) Applicant: Kyocera Corporation
Kyoto-shi Kyoto 612-8501 (JP)

(72) Inventor: MATSUNAGA, Shougo
Kyoto-shi, Kyoto 612-8501 (JP)

(74) Representative: Viering, Jentschura & Partner
mbB
Patent- und Rechtsanwälte
Am Brauhaus 8
01099 Dresden (DE)

(54) **MEASUREMENT MODULE AND MEASUREMENT DEVICE**

(57) A measurement module for measuring a fluid on a flow path defined in an internal space of a flow path section includes a sensor and a reflector. The sensor includes a light emitter and a light receiver. The sensor and the reflector are placeable into a first state in which the sensor and the reflector face each other across the flow path. In the first state, the light receiver receives, in response to the light emitter irradiating the flow path section with light, light scattered by the flow path section, light transmitted through the flow path section and scattered by the fluid on the flow path, and light transmitted through the flow path section and the fluid on the flow path and reflected by the reflector.

FIG. 6

**Description**

TECHNICAL FIELD

The present disclosure relates to a measurement module and a measurement device.

BACKGROUND OF INVENTION

A known device for quantitatively measuring the flow state of a fluid measures the flow rate and the flow velocity of the fluid with an optical method using, for example, a laser blood flowmeter (refer to, for example, WO 2013/153664).

SUMMARY

One or more aspects of the present disclosure are directed to a measurement module and a measurement device.

In an aspect, a measurement module for measuring a fluid on a flow path defined in an internal space of a flow path section includes a sensor and a reflector. The sensor includes a light emitter and a light receiver. The sensor and the reflector are placeable into a first state in which the sensor and the reflector face each other across the flow path. In the first state, the light receiver receives, in response to the light emitter irradiating the flow path section with light, light scattered by the flow path section, light transmitted through the flow path section and scattered by the fluid on the flow path, and light transmitted through the flow path section and the fluid on the flow path and reflected by the reflector.

In an aspect, a measurement module includes a flow path section, a sensor, and a reflector. The flow path section includes, in an internal space of the flow path section, a flow path on which a fluid flows. The sensor includes a light emitter and a light receiver. The reflector faces the sensor across at least a part of the flow path. The light emitter irradiates the flow path section with light. The flow path section includes a light transmitter to transmit the light from the light emitter. The light receiver receives light emitted from the light emitter and scattered by the flow path section, light emitted from the light emitter, transmitted through the light transmitter, and scattered by the fluid on the flow path, and light emitted from the light emitter, transmitted through the light transmitter and the fluid on the flow path, and reflected by the reflector.

In an aspect, a measurement device includes the measurement module according to one of the above aspects, and a processing unit. The light receiver receives light and outputs a signal corresponding to an intensity of the light. The processing unit calculates a quantitative calculation value for a flow state of the fluid based on the signal output from the light receiver.

BRIEF DESCRIPTION OF THE DRAWINGS

FIG. 1 is a schematic front view of a measurement module with an example structure according to a first embodiment, as viewed externally.
FIG. 2 is a schematic plan view of the measurement module with the structure according to the first embodiment, as viewed externally.
FIG. 3 is a schematic exploded view of the measurement module with the structure according to the first embodiment, as viewed externally from the front.
FIG. 4 is a schematic exploded view of the measurement module with the structure according to the first embodiment, as viewed externally from above.
FIG. 5 is a schematic exploded view of the measurement module in FIG. 4, illustrating the cross section taken along line V-V.
FIG. 6 is a schematic diagram of a measurement device with an example structure according to the first embodiment.
FIG. 7 is a functional block diagram of the measurement device according to the first embodiment.
FIG. 8 is a schematic diagram of a measurement device with an example structure in a reference example.
FIG. 9 is a graph showing an example relationship between the flow rate set value and the flow rate calculation value for the measurement devices with the structure in the first embodiment and the structure in the reference example.
FIG. 10 is a schematic front view of a measurement module with an example structure according to a second embodiment, as viewed externally.
FIG. 11 is a schematic plan view of the measurement module with the structure according to the second embodiment, as viewed externally.

FIG. 12 is a schematic front view of the measurement module with the structure according to the second embodiment, as viewed externally.

FIG. 13 is a schematic plan view of the measurement module with the structure according to the second embodiment, as viewed externally.

FIG. 14 is a schematic cross-sectional view of the measurement module in FIG. 13 taken along line XIV-XIV.

FIG. 15 is a schematic front view of a measurement module with an example structure according to a third embodiment, as viewed externally.

FIG. 16 is a schematic exploded view of the measurement module with the structure in FIG. 15, illustrating the cross section corresponding to the cross section taken along line V-V in the measurement module in FIG. 4.

FIG. 17 is a schematic front view of a measurement module with an example structure according to a fourth embodiment, as viewed externally.

FIG. 18 is a schematic cross-sectional view of the measurement module with the structure in FIG. 17, corresponding to the cross section taken along line XIV-XIV in the measurement module in FIG. 13.

FIG. 19 is a schematic front view of a measurement module with an example structure according to a fifth embodiment, as viewed externally.

FIG. 20 is a schematic cross-sectional view of the measurement module with the structure in FIG. 19, corresponding to the cross section taken along line XIV-XIV in the measurement module in FIG. 13.

FIG. 21 is a schematic front view of a measurement module with an example structure according to a sixth embodiment, as viewed externally.

FIG. 22 is a schematic front view of a measurement module with a first example structure according to a seventh embodiment, as viewed externally.

FIG. 23 is a schematic front view of a measurement module with a second example structure according to the seventh embodiment, as viewed externally.

FIG. 24 is a schematic plan view of a measurement module with a first example structure according to an eighth embodiment, as viewed externally.

FIG. 25 is a schematic plan view of a measurement module with a second example structure according to the eighth embodiment, as viewed externally.

FIG. 26 is a schematic plan view of a measurement module with a third example structure according to the eighth embodiment, as viewed externally.

FIG. 27 is a schematic front view of the measurement module with the third structure according to the eighth embodiment, as viewed externally.

FIG. 28 is a schematic exploded view of the measurement module with the third structure according to the eighth embodiment, as viewed externally from above.

FIG. 29 is a schematic exploded view of the measurement module with the third structure according to the eighth embodiment, as viewed externally from the front.

FIG. 30 is a schematic exploded view of the measurement module in FIG. 28, illustrating the cross section taken along line XXX-XXX.

FIG. 31 is a schematic plan view of a measurement module with a fourth example structure according to the eighth embodiment, as viewed externally.

FIG. 32 is a schematic exploded view of the measurement module with the fourth structure according to the eighth embodiment, as viewed externally from above.

FIG. 33 is a schematic exploded view of the measurement module with the fourth structure in FIG. 32 according to the eighth embodiment, illustrating the cross section corresponding to the cross section taken along line XXX-XXX in the measurement module in FIG. 28.

FIG. 34 is a schematic exploded view of a measurement module with a fifth example structure according to the eighth embodiment, as viewed externally from above.

FIG. 35 is a schematic exploded view of a measurement module with a sixth example structure according to the eighth embodiment, as viewed externally from above.

FIG. 36 is a schematic plan view of a measurement module with a first example structure according to a ninth embodiment, as viewed externally.

FIG. 37 is a schematic front view of the measurement module with the first structure according to the ninth embodiment, as viewed externally.

FIG. 38 is a schematic plan view of a measurement module with a second example structure according to the ninth embodiment, as viewed externally.

FIG. 39 is a schematic front view of a measurement module with a third example structure according to the ninth embodiment, as viewed externally.

FIG. 40 is a schematic plan view of the measurement module with the third structure according to the ninth embodiment, as viewed externally.

FIG. 41 is a schematic front view of a measurement module with a first example structure according to a tenth embodiment, as viewed externally.

FIG. 42 is a schematic front view of a measurement module with a second example structure according to the tenth embodiment, as viewed externally.

FIG. 43 is a schematic front view of a measurement module with a third example structure according to the tenth embodiment, as viewed externally.

FIG. 44 is a schematic front view of a measurement module with a fourth example structure according to the tenth embodiment, as viewed externally.

FIG. 45 is a functional block diagram of a measurement device with an example structure according to an eleventh embodiment.

FIG. 46 is a functional block diagram of a measurement device with an example structure according to a twelfth embodiment.

FIG. 47 is a functional block diagram of a measurement system with an example structure in a thirteenth embodiment.

FIG. 48 is a graph showing an example relationship between a flow rate set value and a flow rate calculation value.

DESCRIPTION OF EMBODIMENTS

A known device for quantitatively measuring the flow state of a fluid measures at least one of the flow rate or the flow velocity of the fluid with an optical method using, for example, a laser blood flowmeter. The laser blood flowmeter can calculate the blood flow rate of a living body based on, for example, changes in the wavelength of a laser beam emitted from a laser device as a light emitter onto the living body. The wavelength changes occur due to a Doppler shift resulting from the laser beam scattered by the living body.

More specifically, when a laser beam with a frequency fo is incident on a living body, the laser beam is scattered by the blood flowing through blood vessels (moving particles such as blood cells) and by other fixed tissues (including skin tissue and tissue forming the blood vessels) to be scattered light. The diameter of blood cells ranges from, for example, several micrometers ($\mu$m) to about 20 $\mu$m. Unlike a portion of the scattered light scattered by the other fixed tissues having the frequency fo, a portion of the scattered light scattered by the blood cells has a frequency f changed by a frequency fb to a frequency fo + fb by a Doppler shift corresponding to the movement speed of the particles such as blood cells. This modulated frequency fb is expressed with Formula 1 below, where V is the velocity of the blood flow, $\theta$ is the angle of incidence of a laser beam on the fluid, and $\lambda$ is the wavelength of the laser beam.

$$fb = (2V \times \cos\theta)/\lambda \qquad\qquad (1)$$

Mutual interference between the light with the frequency fo scattered by the fixed tissues and the light with the frequency fo + fb scattered by the moving blood cells allows observation of the difference frequency fb as an optical beat (beat). In other words, a signal (light receiving signal) obtained with a photodetector receiving these two types of light with different frequencies contains a component of a signal (also referred to as an optical beat signal) corresponding to the optical beat resulting from the mutual interference between these two types of light.

The difference frequency fb corresponding to the frequency of the optical beat is far lower than the frequency fo of the laser beam first emitted. For example, light with a wavelength of 780 nm has a frequency of about 400 terahertz (THz), which exceeds the response speed detectable by a normal photodetector. In contrast, the frequency fb of the optical beat (also referred to as an optical beat frequency) is, for example, within the range of about several kilohertz (kHz) to about several tens of kHz and is within a frequency range fully responsive and detectable by a normal photodetector, although the frequency fb changes depending on the movement speed of the blood cells. Thus, the signal (light receiving signal) obtained with the photodetector receiving the light with the frequency fo scattered by the fixed tissues and the light with the frequency fo + fb scattered by the moving blood cells indicates a waveform containing a direct current (DC) component signal (DC signal) on which an intensity modulated signal with the optical beat frequency fb is superimposed. The optical beat signal with the frequency fb is analyzed to calculate the blood flow rate.

For example, a frequency spectrum P(f) for the light receiving signal detected by the photodetector is first calculated using a computation such as a Fourier transform (FFT). The frequency spectrum P(f) is then weighted with the frequency f to calculate a weighted frequency spectrum P(f) $\times$ f. The weighted frequency spectrum P(f) $\times$ f is then integrated within a predetermined frequency range to calculate a first calculation value ($\int\{(f) \times f\}df$). Subsequently, as in Formula 2 below, the first calculation value ($\int\{P(f) \times f\}df$) is divided by a second calculation value ($\int P(f)df$) calculated by integrating the frequency spectrum P(f) within the predetermined frequency range to calculate a mean frequency fm for the optical beat frequency fb.

$$fm = \int \{P(f) \times f\} df / \{\int P(f) df\} \qquad (2)$$

The mean frequency fm may then be used with a predetermined calculation to calculate the blood flow rate of a living body.

The measurement device for measuring the flow rate of a fluid may be used to measure, in addition to blood flowing through a blood vessel, any fluid flowing on a flow path included in a section (also referred to as a flow path section) such as a pipe. When, for example, the laser beam with the frequency fo is incident on the flow path section, the photodetector receives light with an intensity I. The intensity I of the light is expressed with Formula 3 below, where $\alpha 1$ is the amplitude of an electric field component of the light with the frequency fo scattered by the flow path section, $\alpha 2$ is the amplitude of an electric field component of the light with the frequency fo + fb scattered by the particles in the fluid flowing on the flow path, t is time, and $\Delta$ is the phase difference between the electric field component of the light with the frequency fo scattered by the flow path section and the electric field component of the light with the frequency fo + fb scattered by the particles in the fluid flowing on the flow path.

$$I = (\alpha 1^2 + \alpha 2^2)/2 + 2\alpha 1 \alpha 2 \cos\{2\pi fb \times t + \Delta\} \qquad (3)$$

The first term $(\alpha 1^2 + \alpha 2^2)/2$ on the right side of Formula 3 represents a DC component that is constant independently of time t. The second term $2\alpha 1 \alpha 2 \cos\{2\pi fb \times t + \Delta\}$ on the right side of Formula 3 represents an alternating current (AC) component that varies with time t.

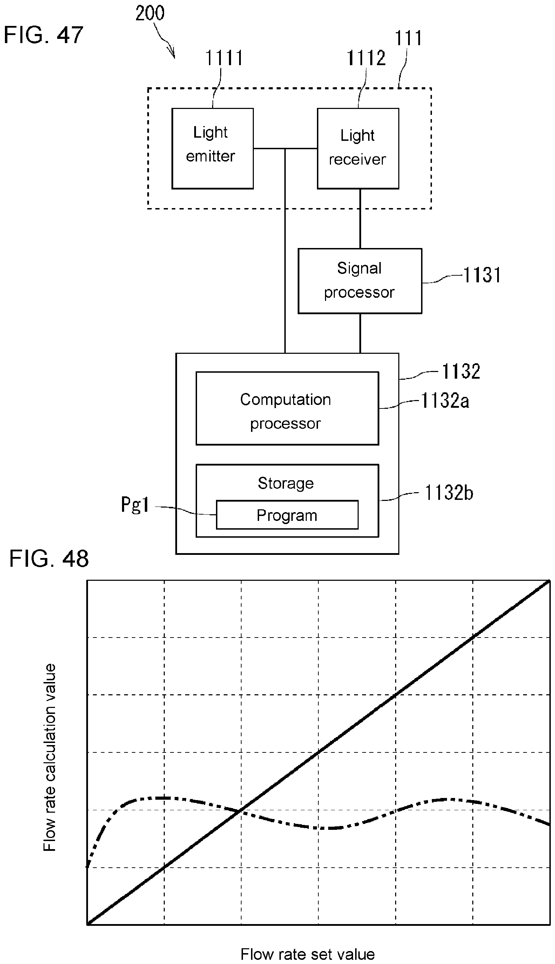

In an example, the flow rate of a fluid flowing through a transparent tube serving as a flow path is measured with a measurement device. The flow rate (also referred to as a flow rate set value) of the fluid flowing on the flow path can be set with, for example, a pump. The flow rate set value is, for example, increased in a stepwise manner. For each flow rate set value, the frequency spectrum P(f) for the optical beat signal, the weighted frequency spectrum P(f) × f, the mean frequency fm, and the flow rate of the fluid are calculated with the measurement device. For the particles contained in the fluid having a relatively large diameter, the flow rate set value and the calculated value of the flow rate (also referred to as a flow rate calculation value) may be substantially in a direct proportional relationship, as illustrated by the thick solid line in FIG. 48.

However, some fluids may contain particles with a relatively small diameter. In this case, for example, the amplitude $\alpha 2$ in Formula 3 is lower, reducing the AC component in the second term on the right side of Formula 3. Thus, the flow rate set value and the flow rate calculation value may deviate greatly from the substantially direct proportional relationship, as illustrated by the thick two-dot-dash line in FIG. 48.

This occurs commonly with any device that measures quantitative values for the flow state of a fluid including at least one of the flow rate or the flow velocity of the fluid, rather than with the measurement device that measures the flow rate of a fluid alone. Measurement modules and devices for quantitative measurement of the flow state of a fluid are thus to be improved to more accurately measure the quantitative values of the flow state of a fluid.

The inventor of the present disclosure and others have developed a technique for improving the accuracy of quantitative measurement of the flow state of a fluid using a measurement module and a measurement device that quantitatively measure the flow state of a fluid.

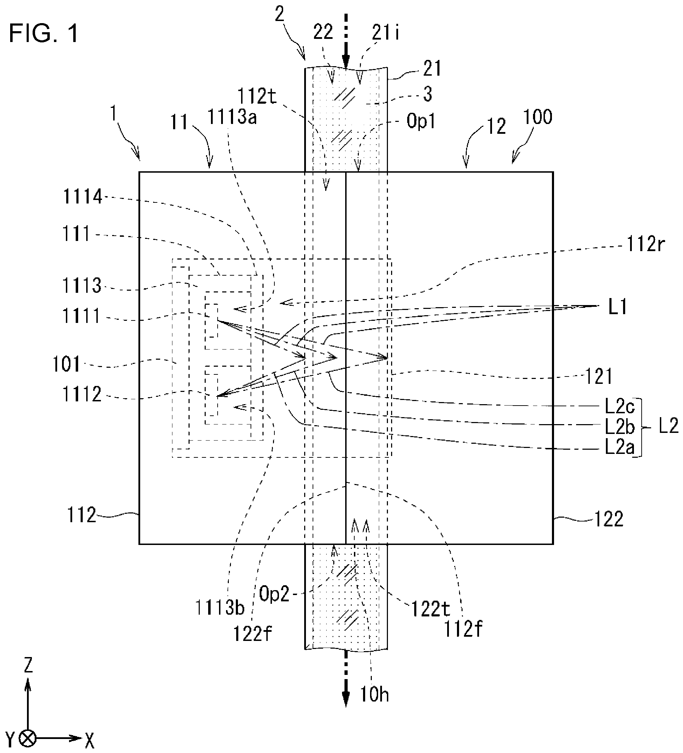

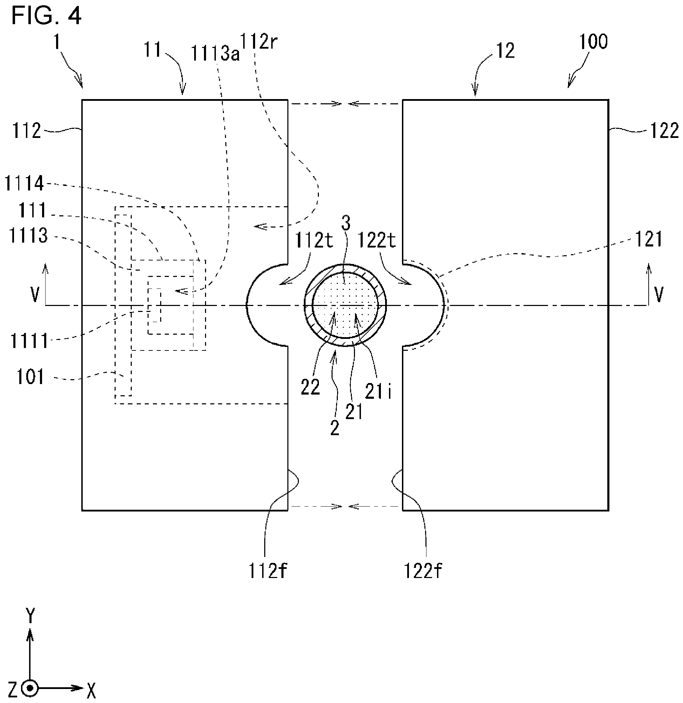

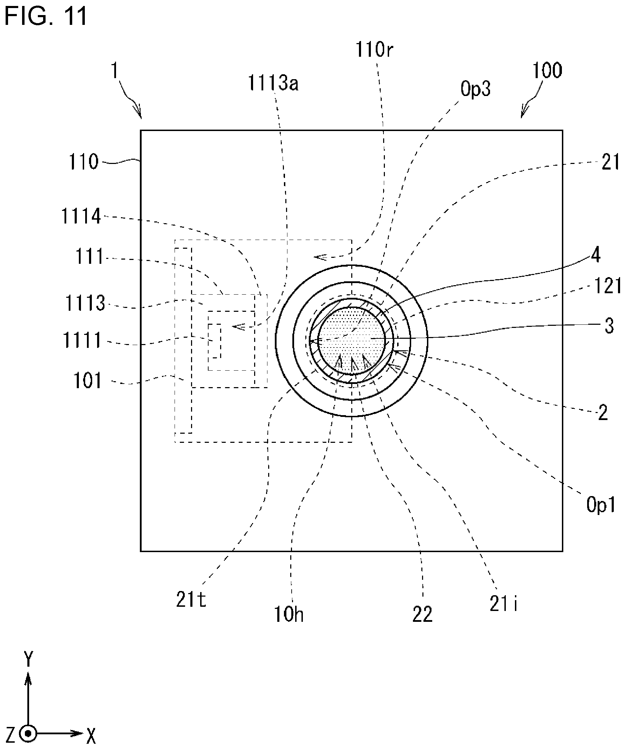

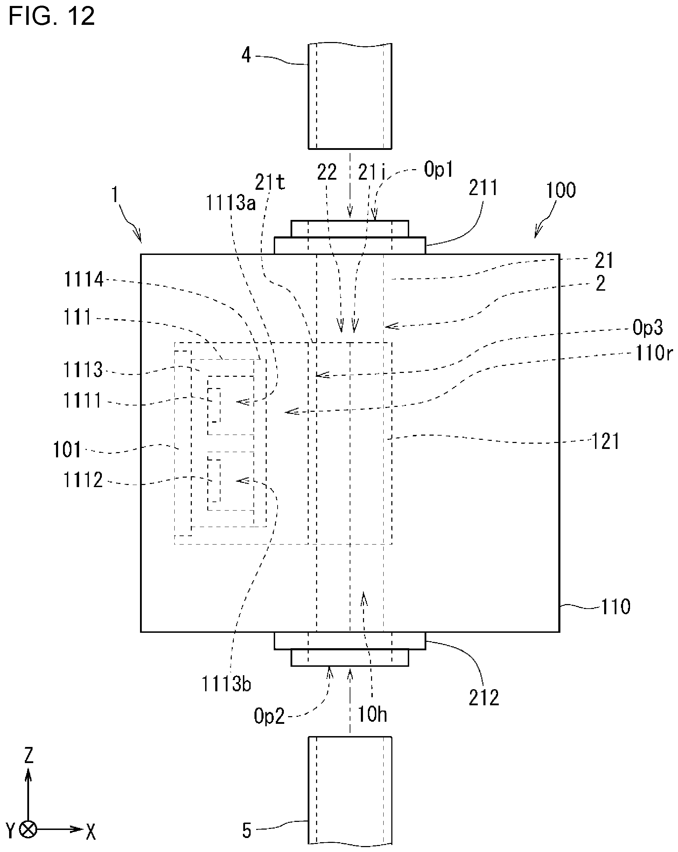

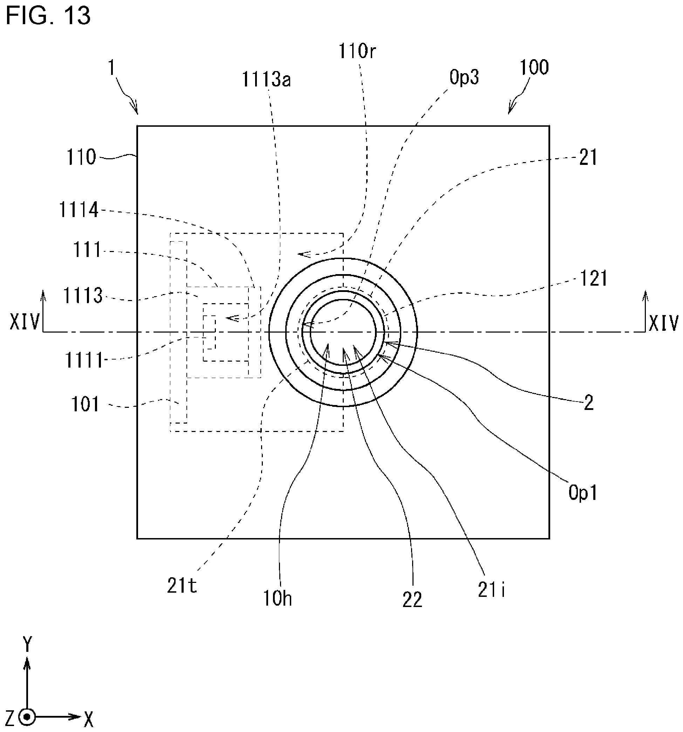

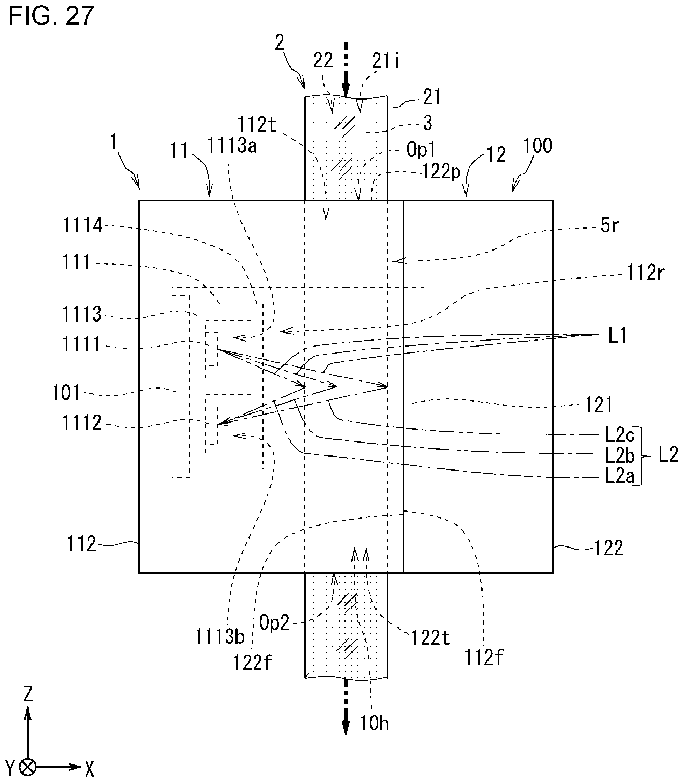

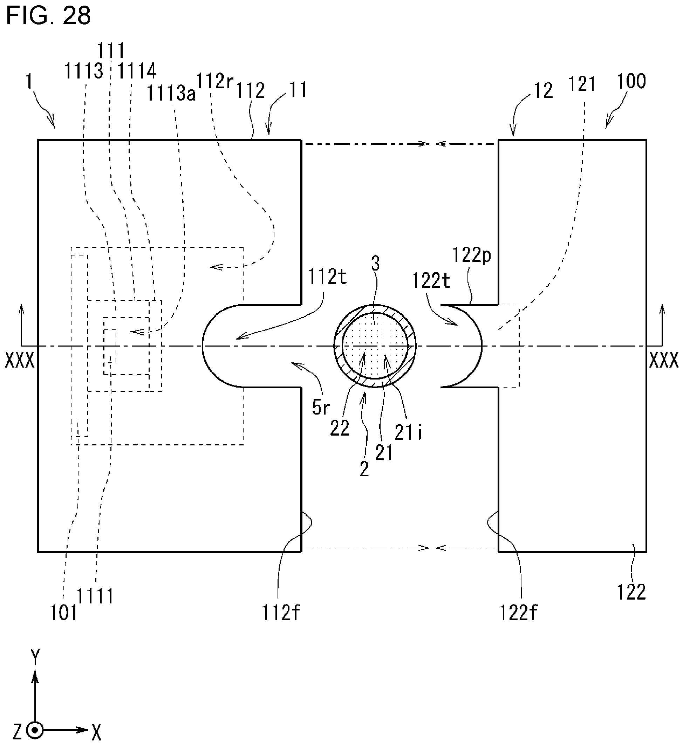

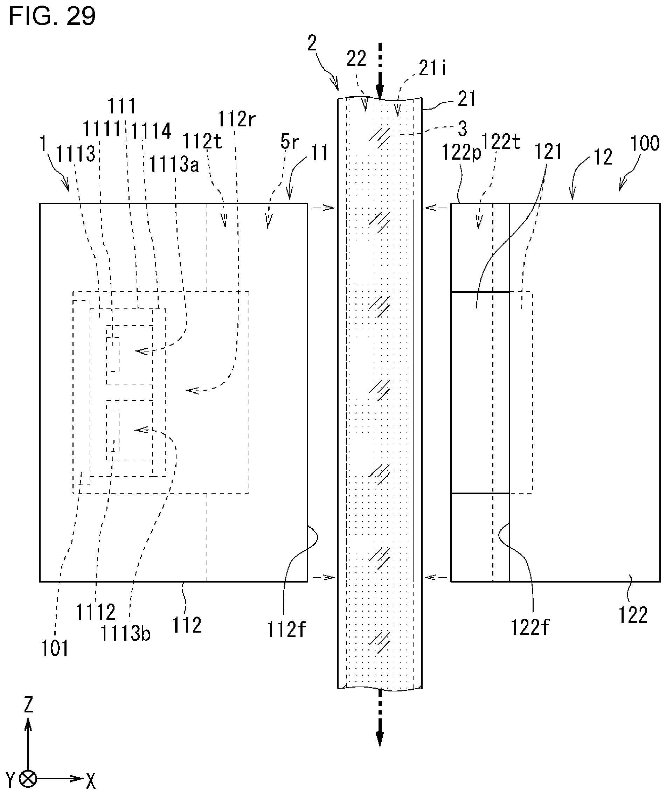

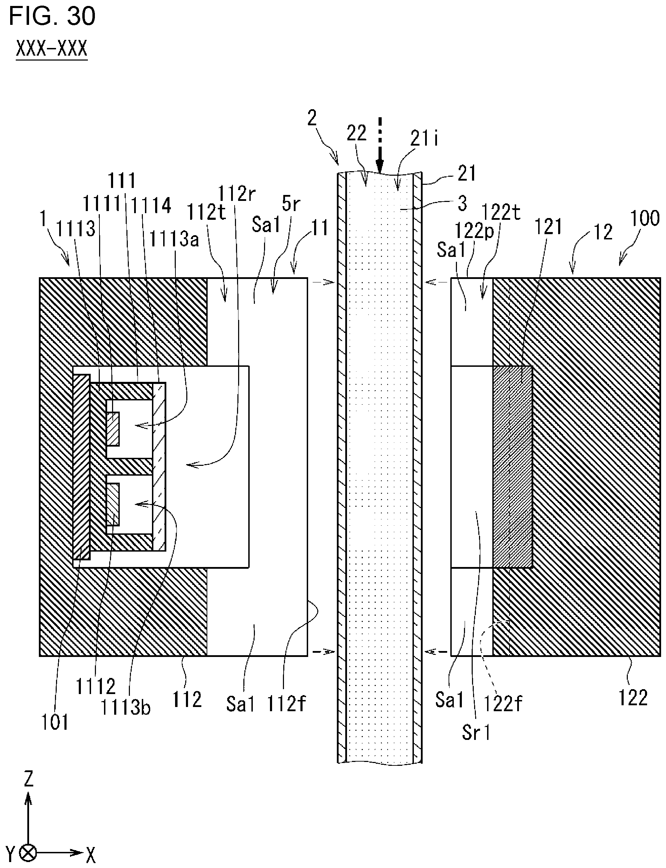

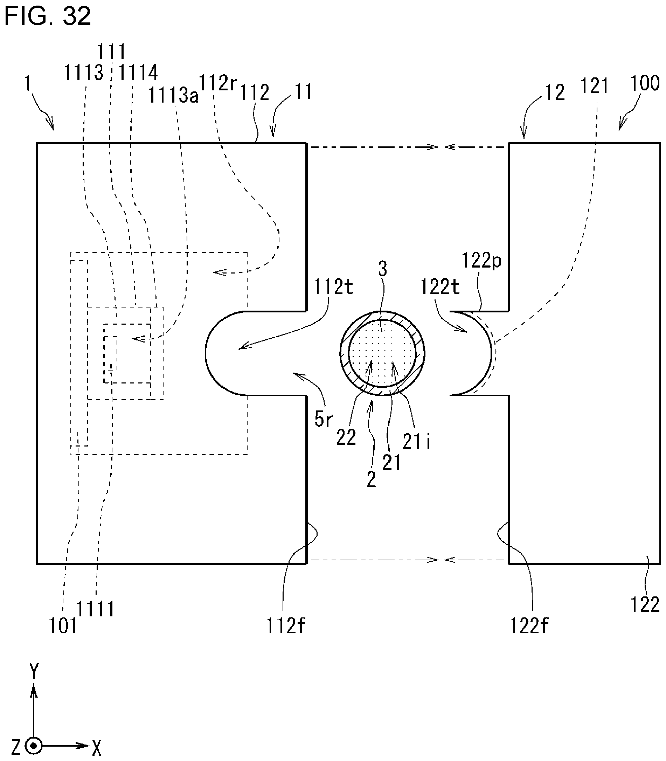

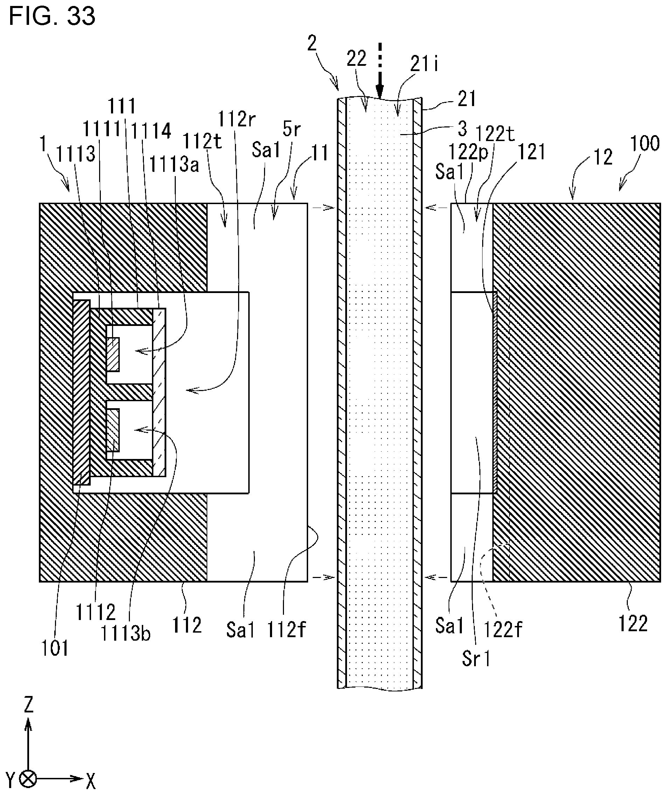

First to thirteenth embodiments associated with the technique will now be described with reference to the drawings. In the drawings, the same reference numerals denote the components having the same or similar structures and functions, and such components will not be described repeatedly. The drawings are schematic. FIGs. 1 to 5 and FIGs. 10 to 44 illustrate the right-handed XYZ coordinate system. In the XYZ coordinate system, the negative Z-direction is the direction in which a fluid 3 flows, the positive X-direction is orthogonal to the negative Z-direction, and the positive Y-direction is orthogonal to both the positive X-direction and the positive Z-direction. In FIGs. 1, 3, 5, 6, 8, 10, 15 to 17, 19, 21 to 23, 27, 29, 30, 33, 37, 39, and 41 to 44, the thick two-dot-dash arrows indicate the direction in which the fluid 3 flows. In FIGs. 1, 6, 8, 10, 15, 17, 19, 21 to 23, 27, 37, and 39, the thin dot-dash line arrows indicate the directions in which irradiation light L1 and coherent light L2 travel.

1. First Embodiment

1-1. Measurement Device

As illustrated in FIGs. 1 to 5, a measurement device 100 according to a first embodiment can quantitatively measure, for example, the flow state of the fluid 3 flowing through an internal space 21i of a section (also referred to as a flow path section) 21 including a flow path 22. The flow path section 21 may include, for example, a tubular object (also referred to as a tubular body) such as a pipe in any device. The tubular body is made of, for example, a light-transmissive material. Examples of the light-transmissive material include glass and a polymer resin. The quantitative values (also referred to as flow quantitative values) indicating the flow state of the fluid 3 may include, for example, at least one of the flow rate or the flow velocity. The flow rate is the quantity of a fluid passing through a flow path per unit time. The quantity of the fluid may be expressed in, for example, volume or mass. The flow velocity is the velocity of the fluid flowing on the flow path. The flow velocity may be expressed with a distance by which the fluid flows per unit time.

The measurement device 100 includes, for example, a measurement module 1. The components of the measurement device 100 and the measurement module 1 (described later) can be manufactured with any known methods as appropriate.

The measurement module 1 quantitatively measures the flow state of the fluid 3 through the flow path 22 in the internal space 21i of the flow path section 21. The measurement module 1 quantitatively measures the flow state of the fluid 3 with, for example, the Doppler effect for light. When, for example, light incident on the fluid 3 is scattered by the fluid 3, the Doppler effect corresponding to the flow of the fluid 3 causes a frequency shift (also referred to as a Doppler shift) of the light corresponding to the movement speed of the fluid 3. The measurement device 100 can detect the doppler shift using, for example, the measurement module 1 to measure the flow quantitative value indicating the flow state of the fluid 3.

Examples of the fluid 3 serving as a target (also referred to as a measurement target) having its flow state quantitatively measured include the fluid 3 that scatters light, a fluid that allows a substance or an object that scatters light (also referred to as a scatter substance or a scatterer) to flow through the fluid. More specifically, examples of the fluid 3 serving as a measurement target include water, blood, printer ink, and gas containing a scatterer such as powder. For a scatter substance or a scatterer flowing with the fluid, the flow rate of the scatter substance or the scatterer may be used as the flow rate of a fluid, and the flow velocity of the scatter substance or the scatterer may be used as the flow velocity of the fluid.

As illustrated in FIGs. 1 to 5, the measurement module 1 according to the first embodiment includes, for example, a sensor 111 and a reflector 121.

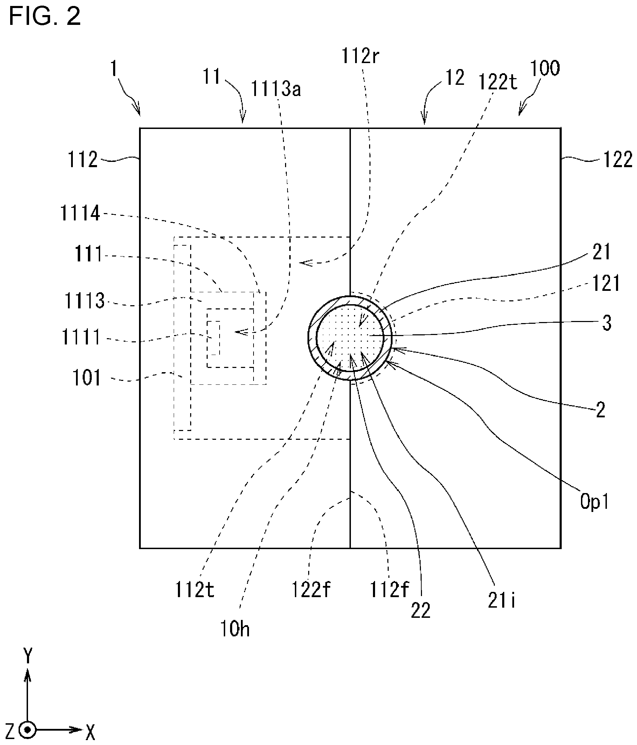

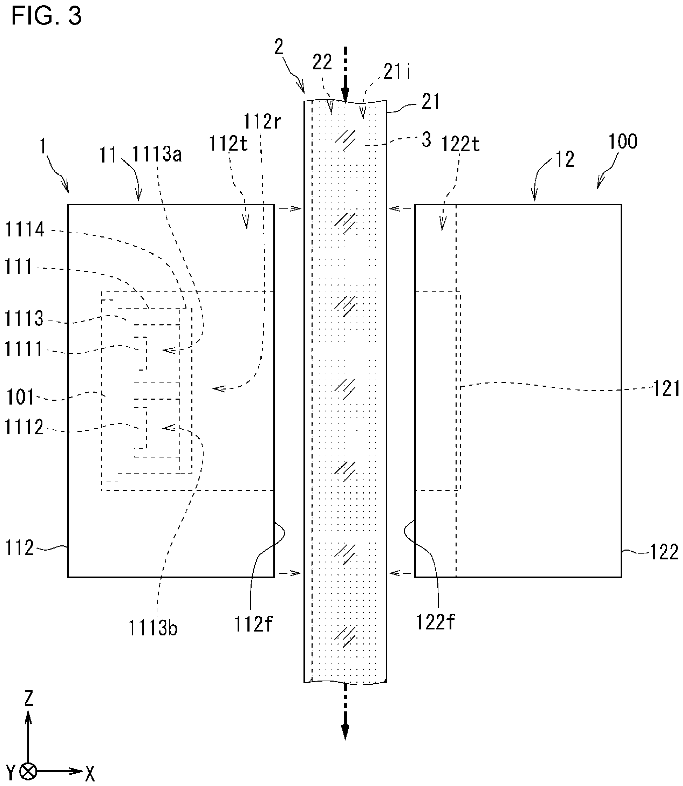

In the measurement module 1 according to the first embodiment, the sensor 111 and the reflector 121 can be, for example, placed to face each other across the flow path 22 (also referred to as being in a first state). For example, the sensor 111 and the reflector 121 each separate from the flow path section 21 as illustrated in FIGs. 3 to 5 (also referred to as being in a second state) can be placed to face each other across the flow path section 21 in the first state as illustrated in FIGs. 1 and 2. The sensor 111 and the reflector 121 can be placed across any tubular body serving as the flow path section 21 to, for example, quantitatively measure the flow state of the fluid 3 through the flow path 22 defined in the internal space 21i of the flow path section 21. The measurement module 1 can thus be installed easily. In the example of FIGs. 1 and 2, the sensor 111 is in the negative X-direction from the flow path 22, and the reflector 121 is in the positive X-direction from the flow path 22.

The sensor 111 includes, for example, a light emitter 1111 and a light receiver 1112.

In the first state, for example, the light emitter 1111 can irradiate, with light (also referred to as irradiation light) L1, an object (also referred to as an irradiation target) 2 that allows the fluid 3 to flow through the internal space 21i. The irradiation target 2 includes at least an object (flow path section) 21 defining a flow path such as a tubular body, and the fluid 3 flowing on the flow path 22. The irradiation light L1 may be, for example, light having a predetermined wavelength for the fluid 3 serving as a measurement target. For the fluid 3 being blood or an isotonic drink, for example, the irradiation light L1 having a wavelength set to about 600 to 900 nanometers (nm) is used. For the fluid 3 being printer ink, for example, the irradiation target 2 is irradiated with light having a wavelength set to about 700 to 1000 nm. The light emitter 1111 may be, for example, a semiconductor laser device such as a vertical-cavity surface-emitting laser (VCSEL).

The light receiver 1112 can receive, for example, coherent light L2 including a portion of irradiation light L1 scattered by the irradiation target 2 and the reflector 121 in the first state. More specifically, for example, the light receiver 1112 can receive, in response to the light emitter 1111 irradiating the flow path section 21 with irradiation light L1, light L2a scattered by the flow path section 21, light L2b transmitted through the flow path section 21 and scattered by the fluid 3 on the flow path 22, and light L2c transmitted through the flow path section 21 and the fluid 3 on the flow path 22 and reflected by the reflector 121. The light receiver 1112 can convert, for example, the received coherent light L2 to an electric signal corresponding to the light intensity. In other words, the light receiver 1112 can receive coherent light L2 and output an electric signal corresponding to the intensity of the coherent light L2.

Coherent light L2 that can be received by the light receiver 1112 includes, for example, scattered light without a Doppler shift from the flow path section 21 and the reflector 121 serving as objects that are stationary around the fluid 3 (also referred to as stationary objects) and scattered light with a Doppler shift with a frequency shift fb from the fluid 3. In other words, coherent light L2 includes, for example, light transmitted through the flow path section 21 and the fluid 3 and reflected by the reflector 121. This can increase, for example, the intensity of the coherent light L2 received by the light receiver 1112.

For example, the intensity I of the coherent light L2 received by the light receiver 1112 is expressed with Formula

4 below, where $\alpha1$ is the amplitude of an electric field component of the scattered light with the frequency fo scattered by the stationary objects, $\alpha2$ is the amplitude of an electric field component of the scattered light with the frequency fo + fb scattered by the particles in the fluid 3 flowing on the flow path 22, t is time, and $\Delta$ is the phase difference between the electric field component of the scattered light with the frequency fo scattered by the stationary objects and the electric field component of the scattered light with the frequency fo + fb scattered by the particles in the fluid 3 flowing on the flow path 22.

$$I = (\alpha1^2 + \alpha2^2)/2 + 2\alpha1\alpha2\cos\{2\pi fb \times t + \Delta\} \qquad (4)$$

For example, the first term $(\alpha1^2 + \alpha2^2)/2$ on the right side of Formula 4 represents a DC component that is constant independently of time t. For example, the second term $2\alpha1\alpha2\cos\{2\pi fb \times t + \Delta\}$ on the right side of Formula 4 represents an AC component of the frequency corresponding to the shift fb, which changes with time t. Such change in the intensity I of coherent light L2 with time (also referred to as a temporal change) can indicate, for example, a beat of the frequency corresponding to a difference (also referred to as a difference frequency) fb between the frequency of the scattered light without a Doppler shift and the frequency of the scattered light with a Doppler shift. Thus, the signal output from the light receiver 1112 and corresponding to the intensity of the coherent light L2 can contain a component of a signal corresponding to the beat (also referred to as a beat signal or an optical beat signal) with respect to the temporal change in the intensity of the coherent light L2. The light receiver 1112 may be, for example, any device that can follow the beat (also referred to as having time resolution) with respect to the temporal change in the intensity of the coherent light L2. The wavelength of light that can be received by the light receiver 1112 can be set in accordance with measurement conditions such as the wavelength of irradiation light L1 and the velocity range of the fluid 3. The light receiver 1112 may be, for example, a silicon (Si) photodiode, a gallium arsenide (GaAs) photodiode, an indium gallium arsenide (InGaAs) photodiode, or a germanium (Ge) photodiode.

In the first embodiment, the scattered light with the frequency fo scattered by a stationary object includes, for example, scattered light with the frequency fo scattered by the reflector 121 in addition to scattered light with the frequency fo scattered by the flow path section 21. This allows the amplitude $\alpha2$ in Formula 4 to be high when, for example, the fluid 3 contains particles with a relatively small diameter and causes a low amplitude $\alpha1$ in Formula 4. This may increase, for example, the AC component of the second term on the right side of Formula 4. In other words, the component of the optical beat signal contained in the signal output from the light receiver 1112 can have, for example, a higher amplitude with a higher intensity I of coherent light L2. This can improve, for example, the measurement accuracy of the quantitative value (flow quantitative value) indicating the flow state of the fluid 3 calculated by analysis on the optical beat signal performed by a processing unit 113 (described later).

The sensor 111 may include, for example, a package 1113. The package 1113 accommodates the light emitter 1111 and the light receiver 1112. In the example of FIGs. 1 to 5, the measurement module 1 includes a board (also referred to as a mounting board) 101 on which the sensor 111 is mounted. The mounting board 101 is, for example, a printed circuit board. The package 1113 in the sensor 111 is located on the mounting board 101. The package 1113 has, for example, a cubic or rectangular parallelepiped external shape. The package 1113 includes, for example, a recess (also referred to as a first recess) 1113a and a recess (also referred to as a second recess) 1113b that are open toward the flow path 22 in the first state. In the example of FIGs. 1 to 5, the first recess 1113a and the second recess 1113b are open in the positive X-direction. The first recess 1113a receives, for example, the light emitter 1111. The second recess 1113b receives, for example, the light receiver 1112. Irradiation light L1 emitted from the light emitter 1111 is incident on the irradiation target 2 through the opening of the first recess 1113a. Coherent light L2 from the irradiation target 2 and the reflector 121 is received by the light receiver 1112 through the opening of the second recess 1113b. The package 1113 may be, for example, a multilayered wiring board made of a ceramic material or an organic material. Examples of the ceramic material include sintered aluminum oxide and sintered mullite. Examples of the organic material include an epoxy resin and a polyimide resin.

As illustrated in FIGs. 1 to 5, the openings of the first recess 1113a and the second recess 1113b in the package 1113 may be covered with a light-transmissive lid 1114. This structure can hermetically seal the light emitter 1111 in the first recess 1113a in the package 1113 and the light receiver 1112 in the second recess 1113b in the package 1113. The lid 1114 may be, for example, a glass plate.

The reflector 121 can reflect, for example, light. For example, the reflector 121 is made of a material with a higher reflectance for irradiation light L1 than a material for the flow path section 21. The material for the reflector 121 may be determined as appropriate in accordance with, for example, the wavelength of irradiation light L1. For example, the reflector 121 may be made of a metal such as aluminum or copper. The reflector 121 is shaped, for example, to align with a portion of the periphery of the flow path section 21 in the positive X-direction in the first state. The reflector 121 may be in the form of a film, a foil member, a plate, or a block. The form of the reflector 121 may be determined as appropriate in accordance with the intensity of irradiation light L1. A reflector 121 having, for example, a higher reflectance for irradiation light L1 may increase the intensity I of coherent light L2 received by the light receiver 1112 and the amplitude of the component of the optical beat signal contained in the signal output from the light receiver 1112. In this case, for example, the amplification ratio of the intensity of the electric signal can be lowered in the process of amplifying the intensity of the electric signal in accordance with the intensity of coherent light L2, thus reducing amplification of noise in the electric signal. This can improve, for example, the measurement accuracy of the quantitative value (flow quantitative value) indicating the flow state of the fluid 3 calculated by analysis on the optical beat signal performed by the processing unit 113 (described later). The higher reflectance of the reflector 121 for irradiation light L1 with the frequency fo may also allow reducing the intensity of irradiation light L1 emitted from the light emitter 1111 within a range in which the measurement accuracy of the quantitative value (flow quantitative value) indicating the flow state of the fluid 3 is maintained. This can reduce, for example, power consumption in the measurement device 100 and the measurement module 1.

The measurement module 1 may include, for example, a first housing 112 and a second housing 122. The first housing 112 and the second housing 122 can be, for example, placed across the flow path section 21 (also referred to as being placed in a third state) with the sensor 111 and the reflector 121 facing each other across the flow path 22 in the first state.

The first housing 112 includes, for example, a recess (also referred to as a third recess) 112r that surrounds the sensor 111 from a position opposite to the flow path section 21 in the third state. The first housing 112 may be, for example, in any external shape such as cubic, rectangular, or semicylindrical. The third recess 112r is, for example, open toward the flow path 22 in the third state. In the example of FIGs. 1 to 5, the third recess 112r includes an opening that is open in the positive X-direction. In the third state, for example, the third recess 112r receives the sensor 111. In the third state, for example, the third recess 112r may receive the mounting board 101 on which the sensor 111 is mounted. The sensor 111 may be, for example, fixed to the first housing 112 to be an integral piece (also referred to as a first section) 11. The mounting board 101 on which the sensor 111 is mounted may be screwed, crimped, or adhered to the first housing 112 within the third recess 112r. The measurement module 1 thus includes, for example, the first section 11 including the sensor 111 and the first housing 112. This facilitates, for example, placement of the sensor 111 and the first housing 112 with respect to the flow path section 21.

The first housing 112 includes, for example, a surface (also referred to as a first surface) 112f located around the opening of the third recess 112r and extending along the second housing 122 in the third state. The first surface 112f may be, for example, in contact with or adjacent to the second housing 122 in the third state. In the example of FIGs. 1 to 5, the first surface 112f is the surface of the first housing 112 facing in the positive X-direction. The first housing 112 is shaped, for example, to extend along the outer surface of the flow path section 21 in the third state. The first housing 112 includes, for example, the third recess 112r and a first groove 112t on the first surface 112f. In the example of FIGs. 1 to 5, the third recess 112r is substantially in the middle of the first surface 112f, and the first groove 112t extends straight in the positive Z-direction and the negative Z-direction from the third recess 112r. The first groove 112t is shaped to, for example, receive a portion of the flow path section 21 in the negative X-direction in the third state. For example, the first groove 112t may include a semicylindrical internal space, and the flow path section 21 may be cylindrical.

The second housing 122 includes, for example, a surface (also referred to as a second surface) 122f that is placeable along the first surface 112f in the third state. The second surface 122f may be, for example, in contact with or adjacent to the first surface 112f in the third state. In the example of FIGs. 1 to 5, the second surface 122f is the surface of the second housing 122 facing in the negative X-direction. The second housing 122 surrounds, for example, the reflector 121 from a position opposite to the flow path section 21. The second housing 122 may be, for example, in any external shape such as cubic, rectangular, or semicylindrical.

The second housing 122 is shaped, for example, to extend along the outer surface of the flow path section 21 in the third state. The second housing 122 includes, for example, a second groove 122t on the second surface 122f. The second groove 122t extends, for example, in one direction along the second surface 122f. In the example of FIGs. 1 to 5, the second groove 122t extends straight in Z-direction substantially in the middle of the second surface 122f in Y-direction. The second groove 122t is shaped to, for example, receive a portion of the flow path section 21 in the positive X-direction in the third state. For example, the second groove 122t may include a semicylindrical internal space, and the flow path section 21 may be cylindrical. The reflector 121 is, for example, located along the bottom (inner surface) of the second groove 122t. In the example of FIGs. 1 to 5, the reflector 121 is semicylindrical and extends along the bottom of the second groove 122t. The reflector 121 may be, for example, fixed to the second housing 122 to be an integral piece (also referred to as a second section) 12. For example, a film-like reflector 121 may be formed along the bottom of the second groove 122t by dry deposition such as vapor deposition or sputtering or by wet deposition such as plating or coating. The reflector 121 may be attached along the bottom of the second groove 122t with, for example, an adhesive or adhesive tape. The measurement module 1 thus includes, for example, the second section 12 including the reflector 121 and the second housing 122. This facilitates, for example, placement of the reflector 121 and the second housing 122 with respect to the flow path section 21.

In this example, the first section 11 including the first housing 112 and the second section 12 including the second housing 122 that are separate from each other as illustrated in FIGs. 3 to 5 are placed across the flow path section 21 as in the third state as illustrated in FIGs. 1 and 2. This allows the sensor 111 and the reflector 121 separate from the flow path 22 in the second state to face each other across the flow path 22 in the first state. The measurement module 1 can thus be installed easily with respect to any tubular body serving as the flow path section 21 to, for example, quantitatively measure the state of the fluid 3 flowing through the internal space 21i of the flow path section 21. In the third state, the first section 11 and the second section 12 may be connected by adhesion using, for example, an adhesive or adhesive tape, or by fastening or clamping using other members.

The first housing 112 and the second housing 122 include, for example, a first opening Op1 and a second opening Op2 in the third state. The first opening Op1 is, for example, at an end (also referred to as a first end) of the flow path section 21 in the longitudinal direction (e.g., positive Z-direction). The flow path section 21 extends through the first opening Op1. The second opening Op2 is, for example, at the end (also referred to as a second end) of the flow path section 21 opposite to the first end in the longitudinal direction (e.g., positive Z-direction). The flow path section 21 extends through the second opening Op2. In the example of FIGs. 1 and 2, the measurement module 1 includes a through-hole 10h defined by the first groove 112t and the second groove 122t. The through-hole 10h includes the first opening Op1 in the positive Z-direction and the second opening Op2 in the negative Z-direction. The tubular body serving as the flow path section 21 extends, for example, through the through-hole 10h.

The first housing 112 and the second housing 122 may have, for example, higher light-shielding than the flow path section 21. In this case, light from outside the measurement module 1 is less likely to enter the light receiver 1112. This can improve, for example, the accuracy of quantitative measurement of the flow state of the fluid 3 on the flow path 22. The first housing 112 and the second housing 122 may be made of, for example, a black resin having high light-shielding for visible light. The first housing 112 and the second housing 122 may be made of, for example, a metal material or a ceramic material having high light-shielding for visible light.

The reflector 121 may be, for example, distant from the first opening Op1 in the through-hole 10h as in FIG. 1. In this case, light entering the through-hole 10h from outside the measurement module 1 through the first opening Op1 is less likely to reach the light receiver 1112 due to reflection by the reflector 121. Thus, light from outside the measurement module 1 is less likely to enter the light receiver 1112. This can improve, for example, the accuracy of quantitative measurement of the flow state of the fluid 3 on the flow path 22. The reflector 121 may be, for example, distant from the second opening Op2 in the through-hole 10h as in FIG. 1. In this case, light entering the through-hole 10h from outside the measurement module 1 through the second opening Op2 is less likely to reach the light receiver 1112 due to the reflection by the reflector 121. Thus, light from outside the measurement module 1 is less likely to enter the light receiver 1112. This can improve, for example, the accuracy of quantitative measurement of the flow state of the fluid 3 on the flow path 22. The portion of the inner surface of the through-hole 10h excluding the reflector 121 may have, for example, reflectance for light lower than the reflectance of the reflector 121 for irradiation light L1.

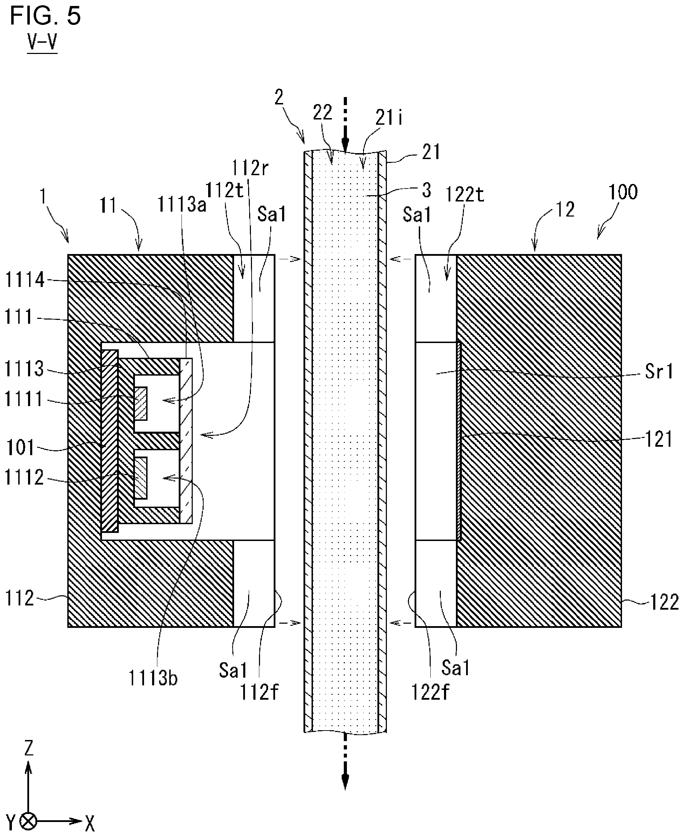

In other words, as illustrated in, for example, FIG. 5, the reflector 121 includes a surface (also referred to as a reflective surface) Sr1 that faces the sensor 111 across the flow path 22 in the first state. The inner surface of the first groove 112t and a portion of the inner surface of the second groove 122t not receiving the reflector 121 may define, for example, a surface (also referred to as a low reflective surface) Sa1 having lower reflectance for irradiation light L1 than the reflective surface Sr1. The low reflective surface Sa1 may be made of, for example, a black material such as a black resin. The low reflective surface Sa1 may be made of, for example, a material for the first housing 112 and the second housing 122, or may be a black film extending along the bottoms of the first groove 112t and the second groove 122t. The black film may be formed by, for example, deposition of a black resin by coating or other techniques. The black film may be attached along the bottoms of the first groove 112t and the second groove 122t with, for example, an adhesive or adhesive tape.

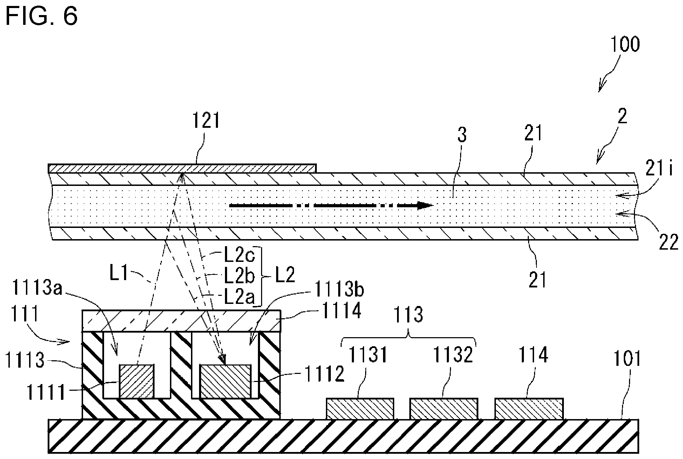

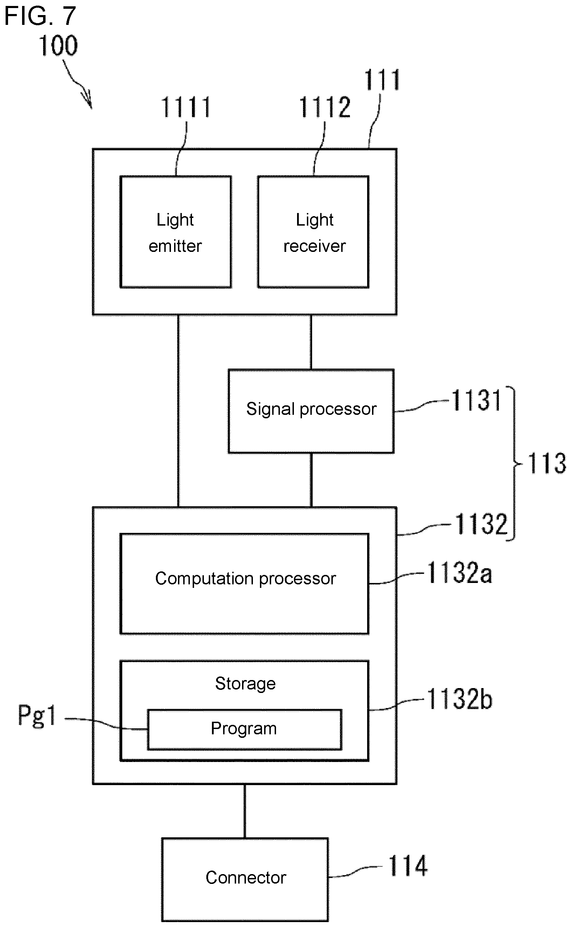

As illustrated in FIGs. 6 and 7, the measurement device 100 includes the processing unit 113 in addition to the measurement module 1 described above. As illustrated in the example of FIG. 6, the sensor 111, the processing unit 113, and a connector 114 may be mounted on the mounting board 101. The mounting board 101 electrically connects, for example, the sensor 111 to the processing unit 113 and the processing unit 113 to the connector 114. For example, the third recess 112r on the first housing 112 may receive the mounting board 101 on which the sensor 111, the processing unit 113, and the connector 114 are mounted.

The processing unit 113 can calculate, for example, a quantitative calculation value for the flow state of the fluid 3 based on the electric signal output from the light receiver 1112. The processing unit 113 may control, for example, the measurement device 100. The processing unit 113 includes multiple electronic components including active elements such as a transistor or a diode and a passive element such as a capacitor. The connector 114 can electrically connect, for example, the processing unit 113 to external devices. The connector 114 and external devices may be electrically connected with, for example, wiring routed through the first housing 112. Multiple electronic components may be, for example, integrated into one or more integrated circuits (ICs) or large-scale integration circuits (LSIs), or multiple ICs or LSIs may be further integrated to implement various functional units including the processing unit 113 and the connector 114. Multiple electronic components serving as the processing unit 113 and the connector 114 are mounted on, for example, the mounting board 101. The package 1113 is thus, for example, electrically connected to the processing unit 113, and the processing unit 113 is electrically connected to the connector 114.

The processing unit 113 includes, for example, a signal processor 1131 and an information processor 1132.

The signal processor 1131 can perform, for example, various processes on the electric signal received from the light receiver 1112. Examples of the various processes may include conversion of an electric signal to a voltage, amplification of the strength of an electric signal, conversion of an analog signal to a digital signal, and separation of an electric signal into an AC component and a DC component. For example, the signal processor 1131 may separate the electric signal output from the light receiver 1112 into DC and AC components and then amplify the AC component signal (also referred to as an AC signal). In this case, the various processes performed by the signal processor 1131 may include, for example, conversion of an electric signal to a voltage, separation of an electric signal into AC and DC components (also referred to as AC-DC separation), amplification of the AC signal, and conversion of an analog signal to a digital signal. The signal processor 1131 may include, for example, circuits such as a current-voltage conversion circuit (I-V conversion circuit), an AC-DC separation circuit (AC-DC decoupling circuit), an amplifier circuit, and an analog-to-digital conversion circuit (AD conversion circuit). The signal processor 1131 can thus perform, for example, processes such as AC-DC separation, amplification, and AD conversion on an analog electric signal received from the light receiver 1112, and then output a digital signal to the information processor 1132. The signal processor 1131 may perform, for example, processes such as amplification and AD conversion on an analog electric signal received from the light receiver 1112, and then output a digital signal to the information processor 1132.

The information processor 1132 includes, for example, a computation processor 1132a and a storage 1132b.

The computation processor 1132a includes, for example, a processor serving as an electric circuit. The processor may include, for example, one or more processors, a controller, a microprocessor, a microcontroller, an application-specific integrated circuit (ASIC), a digital signal processor, a programmable logic device, a combination of any of these devices or components, or a combination of any other known devices or components.

The storage 1132b includes, for example, a random-access memory (RAM) and a read-only memory (ROM). The storage 1132b stores, for example, firmware containing a program Pg1. The computation processor 1132a can perform, for example, computation or processing on one or more pieces of data in accordance with the firmware stored in the storage 1132b. In other words, for example, the computation processor 1132a executing the program Pg1 allows implementation of the various functions of the measurement device 100. The information processor 1132 can thus control, for example, the operation of the light emitter 1111 and the light receiver 1112.

The frequency and the signal strength of an electric signal output from, for example, the light receiver 1112 vary depending on the Doppler effect for light. Thus, the frequency spectrum P(f) showing the relationship between the frequency and the strength of the electric signal varies in accordance with the flow quantitative value (the flow rate or the flow velocity) of the fluid 3. The information processor 1132 can thus perform, for example, computation to quantitatively measure the flow state of the fluid 3 based on the electric signal output from the light receiver 1112 and then processed by the signal processor 1131 with the computation processor 1132a.

More specifically, the computation processor 1132a first calculates, using computation such as a Fourier transform (FFT), a power spectrum (also referred to as a first frequency spectrum) P(f) showing the distribution of the signal strength for each frequency for a temporal change in the signal strength of, for example, the signal output from the light receiver 1112. In other words, the computation processor 1132a calculates, for example, the first frequency spectrum P(f) for the temporal change in the strength of the signal output from the light receiver 1112. For example, the computation processor 1132a calculates the frequency spectrum P(f) for a change over time (temporal change) in the strength of a signal obtained by amplification of the signal output from the light receiver 1112 with the signal processor 1131 or an AC signal obtained by AC-DC separation and amplification of the signal output from the light receiver 1112 with the signal processor 1131. The first frequency spectrum P(f) is, for example, obtained by performing analysis with a computation such as a Fourier transform on the temporal change in the strength of an AC component of the signal output from the signal processor 1131. The frequency range in the first frequency spectrum P(f) may be set based on, for example, the sampling rate in the AD conversion circuit.

The computation processor 1132a weights the first frequency spectrum P(f) with a frequency f to calculate a weighted frequency spectrum (also referred to as a second frequency spectrum) $P(f) \times f$.

Subsequently, the computation processor 1132a calculates, for example, the integrated value ($\int\{P(f) \times f\}df$) of the strength for the second frequency spectrum $P(f) \times f$ and the integrated value ($\int P(f)df$) of the strength for the first frequency spectrum P(f).

The computation processor 1132a then calculates, for example, the mean frequency fm for the difference frequency fb by dividing the integrated value ($\int\{P(f) \times f\}df$) of the strength for the second frequency spectrum $P(f) \times f$ by the integrated value ($\int P(f)df$) of the strength for the first frequency spectrum P(f).

The computation processor 1132a can then calculate the quantitative value (flow quantitative value) indicating the flow state of the fluid 3 based on, for example, the mean frequency fm calculated as described above. This allows, for example, the measurement device 100 to quantitatively measure the flow state of the fluid 3 flowing through the internal space 21i of the flow path section 21 including the flow path 22.

For example, the computation processor 1132a can calculate the quantitative value (flow quantitative value) for the flow of the fluid 3 based on the mean frequency fm and prepared calibration data (also referred to as a calibration curve). For example, with the calibration data about the flow rate of the fluid 3 being prepared, the flow rate of the fluid 3 may be calculated based on the mean frequency fm and the calibration curve for the flow rate serving as the flow quantitative value. For example, with the calibration data about the flow velocity of the fluid 3 being prepared, the flow velocity of the fluid 3 may be calculated based on the mean frequency fm and the calibration curve for the flow velocity serving as the flow quantitative value. This allows calculation of at least one of the flow rate or the flow velocity of the fluid 3.

For example, the calibration data may be prestored in the storage 1132b before the flow quantitative value of the fluid 3 is measured. The calibration data may be stored in the form of, for example, a functional formula or a table.

The calibration data may be prepared by, for example, the measurement device 100 calculating the mean frequency fm of the fluid 3 flowing on the flow path section 21 at a known flow quantitative value. The calculation of the mean frequency fm performed by the measurement device 100 involves irradiating, with the light emitter 1111, the irradiation target 2 with irradiation light L1, receiving, with the light receiver 1112, coherent light L2 including light scattered by the irradiation target 2 and the reflector 121, and calculating the mean frequency fm with the computation processor 1132a. The measurement device 100 may calculate, for example, the mean frequency fm of the fluid 3 flowing on the flow path section 21 at a known flow quantitative value, and derives calibration data based on the relationship between the known flow quantitative value and the calculated mean frequency fm. More specifically, for example, the derived calibration data may include an operation expression (calibration curve) including the mean frequency fm as a parameter.

For example, the calibration curve is expressed with Formula 5 including a coefficient a, a coefficient b, and a constant c, where y is the flow quantitative value and x is the mean frequency fm.

$$y = a \times x^2 + b \times x + c \qquad (5)$$

When, for example, the mean frequency fm of the fluid 3 flowing on the flow path section 21 at a known flow quantitative value y1 is calculated as a value x1, the mean frequency fm of the fluid 3 flowing on the flow path section 21 at a known flow quantitative value y2 is calculated as a value x2, and the mean frequency fm of the fluid 3 flowing on the flow path section 21 at a known flow quantitative value y3 is calculated as a value x3, Formulas 6, 7, and 8 below are obtained.

$$y1 = a \times x1^2 + b \times x1 + c \qquad (6)$$

$$y2 = a \times x2^2 + b \times x2 + c \qquad (7)$$

$$y3 = a \times x3^2 + b \times x3 + c \qquad (8)$$

The coefficients a and b and the constant c are calculated from Formulas 6, 7, and 8. The calculated coefficients a and b and constant c are substituted into Formula 5 to obtain the calibration data indicating the calibration curve.

The functional formula representing the calibration curve may be, for example, expressed in a polynomial expression including an n-th order (where n is a natural number greater than or equal to 2) term including y as the flow quantitative value and x as the mean frequency fm being a variable. The functional formula representing the calibration curve may include, for example, at least one term selected from the term of logarithm and the term of exponentiation of the variable x as the mean frequency fm.

For example, the flow rate of the fluid 3 on the flow path 22 is calculated using the measurement device 100 with the above structure (also referred to as a flow rate calculation value) while the flow rate of the fluid 3 on the flow path 22 is increased in a stepwise manner with, for example, a pump (also referred to as a flow rate set value). In this case, the flow rate set value and the flow rate calculation value may be substantially in a direct proportional relationship. The flow rate set value and the flow rate calculation value may remain in the substantially direct proportional relationship when, for example, the particles contained in the fluid 3 have a smaller diameter. For example, the flow velocity of the fluid 3 on the flow path 22 may be calculated using the measurement device 100 with the above structure (also referred to as a flow velocity calculation value) while the flow velocity of the fluid 3 on the flow path 22 is increased in a stepwise manner with, for example, a pump (also referred to as a flow velocity set value). In this case, the flow velocity set value and the flow velocity calculation value may be substantially in a direct proportional relationship. The flow velocity set value and the flow velocity calculation value may remain in the substantially direct proportional relationship when, for example, the particles contained in the fluid 3 have a smaller diameter. The measurement device 100 thus allows more accurate quantitative measurement of the flow state of the fluid 3. 1-2. Specific Examples of Measurement Results with Measurement Devices

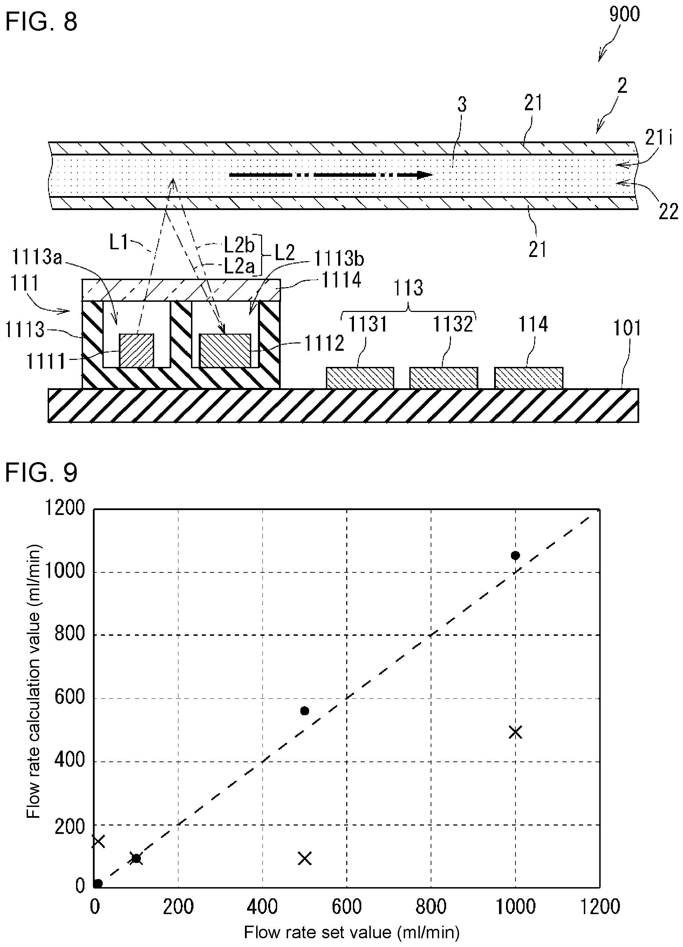

Specific calculation results of the flow calculation values using the measurement device 100 including the reflector 121 in the first state will now be described. In the first state, the reflector 121 faces the sensor 111 across the flow path 22 as described above. As illustrated in FIG. 8, a measurement device 900 in a reference example eliminating the reflector 121 from the measurement device 100 is also used. The flow rate calculation values calculated with the measurement device 900 are also described as results in reference examples. In the example, a cylindrical tube made of a tetrafluoroethylene-perfluoroalkyl vinyl ether copolymer or a perfluoroalkoxy (PFA) fluoropolymer with a diameter of 3 millimeters (mm) was used as a pipe defining the flow path section 21. The distance between the lid 1114 of the sensor 111 and the pipe was set to about 2 mm. A VCSEL, which emits light with a wavelength of 850 nm and an intensity of 1.0 milliwatt (mW), was used as the light emitter 1111. A Si photodiode was used as the light receiver 1112. Copper foil as the reflector 121 was placed on a portion of the outer peripheral surface of the pipe opposite to the sensor 111. The fluid 3 was prepared by mixing 50 grams (g) of amino acids powder with an average particle size of 20 $\mu$m into 100 milliliters (ml) of pure water. The flow rate (flow rate set value) of the fluid 3 flowing on the flow path 22 in the pipe was adjusted to 10 milliliters per minute (ml/min), 100 ml/min, 500 ml/min, and 1000 ml/min with a pump to calculate flow rate calculation values with the measurement device 100.

In FIG. 9, the black dots indicate the relationship between the flow rate set value and the flow rate calculation value calculated with the measurement device 100 according to the first embodiment. In FIG. 9, the cross marks indicate the relationship between the flow rate set value and the flow rate calculation value calculated with the measurement device 900 in the reference example.

As shown in FIG. 9, the relationship between the flow rate set value and the flow rate calculation value calculated with the measurement device 900 in the reference example deviates greatly from a direct proportional relationship. In contrast, with the measurement device 100 according to the first embodiment, the relationship between the flow rate set value and the calculated flow rate calculation value is substantially in a direct proportional relationship.

This indicates that the reflector 121 facing the sensor 111 across the flow path 22 improves the accuracy of quantitative measurement of the flow state of the fluid 3. The reflector 121 seems to increase the intensity I of the coherent light L2 received by the light receiver 1112 in response to the light emitter 1111 irradiating the flow path section 21 with irradiation light L1 and improve the measurement accuracy of the flow quantitative value indicating the flow state of the fluid 3. More specifically, the increase in the intensity of coherent light L2 seems to increase the amplitude of the component of the optical beat signal contained in the signal output from the light receiver 1112 and improve the measurement accuracy of the flow quantitative value indicating the flow state of the fluid 3 calculated by analysis on the optical beat signal. 1-3. Overview of First Embodiment

As described above, the measurement device 100 and the measurement module 1 according to the first embodiment include, for example, the reflector 121 and the sensor 111 including the light emitter 1111 and the light receiver 1112. The sensor 111 and the reflector 121 can be placed into the first state in which the sensor 111 and the reflector 121 face each other across the flow path section 21. In the first state, for example, the light receiver 1112 can receive, in response to the light emitter 1111 irradiating the flow path section 21 with irradiation light L1, light L2a scattered by the flow path section 21, light L2b transmitted through the flow path section 21 and scattered by the fluid 3 on the flow path 22, and light L2c transmitted through the flow path section 21 and the fluid 3 on the flow path 22 and reflected by the reflector 121. This may increase, for example, the intensity I of the coherent light L2 received by the light receiver 1112 in response to the light emitter 1111 irradiating the flow path section 21 with irradiation light L1 and the amplitude of the optical beat signal component in the signal output from the light receiver 1112. This may thus improve, for example, the measurement accuracy of the quantitative value (flow quantitative value) indicating the flow state of the fluid 3 calculated by analysis on the optical beat signal. Thus, the accuracy of quantitative measurement of the flow state of the fluid 3 on the flow path 22 may be improved.

## 2. Other Embodiments

The present disclosure is not limited to the first embodiment and may be changed or varied in various manners without departing from the spirit and scope of the present disclosure.

## 2-1. Second Embodiment

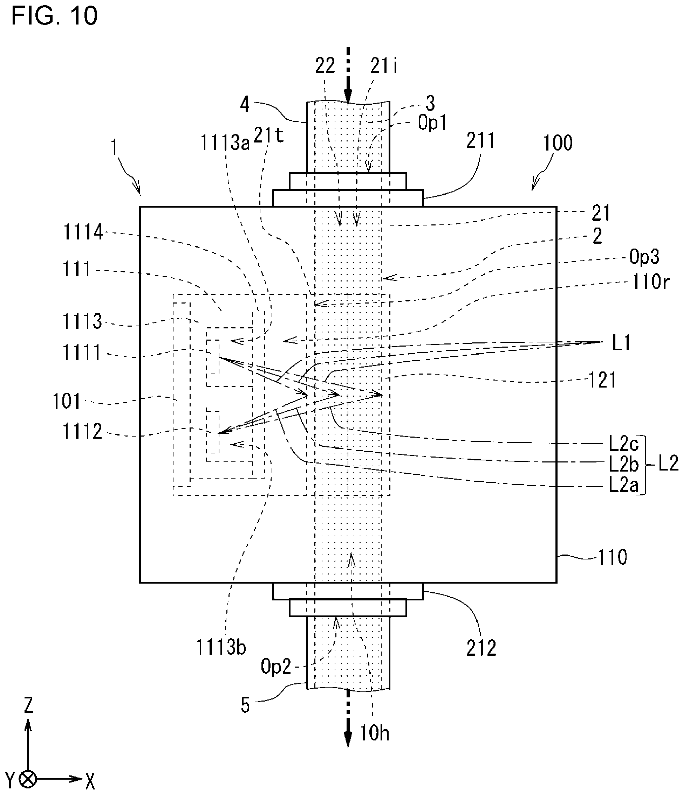

In the first embodiment, for example, the measurement module 1 for quantitatively measuring the flow state of the fluid 3 may include the flow path section 21 that includes, in its internal space 22i, the flow path 22 on which the fluid 3 flows, as illustrated in FIGs. 10 to 14. The measurement module 1 includes, for example, the sensor 111 and the reflector 121. The sensor 111 includes the light emitter 1111 that irradiates the flow path section 21 and the light receiver 1112. The reflector 121 faces the sensor 111 across at least a part of the flow path 22. The flow path section 21 may further include, for example, a part (also referred to as a light transmitter) 21t that transmits light (irradiation light L1) emitted from the light emitter 1111. The light receiver 1112 may receive, for example, light L2a emitted from the light emitter 1111 and scattered by the flow path section 21, light L2b emitted from the light emitter 1111, transmitted through the light transmitter 21t, and scattered by the fluid 3 on the flow path 22, and light L2c emitted from the light emitter 1111, transmitted through the light transmitter 21t and the fluid 3 on the flow path 22, and reflected by the reflector 121.

With this structure, for example, the coherent light L2 received by the light receiver 1112 in response to the light emitter 1111 irradiating the flow path section 21 with irradiation light L1 includes light L2a scattered by the flow path section 21, light L2b scattered by the fluid 3, and light L2c reflected by the reflector 121. This may increase, for example, the intensity I of the coherent light L2 received by the light receiver 1112 and the amplitude of the optical beat signal component in the signal output from the light receiver 1112. This may thus improve, for example, the measurement accuracy of the quantitative value (flow quantitative value) indicating the flow state of the fluid 3 calculated by analysis on the optical beat signal. Thus, the accuracy of quantitative measurement of the flow state of the fluid 3 on the flow path 22 may be improved.

A measurement module 1 with an example structure according to a second embodiment illustrated in FIGs. 10 to 14 is based on the structure of the measurement module 1 according to the first embodiment illustrated in FIGs. 1 to 5. The measurement module 1 with the structure according to the second embodiment includes a housing 110 corresponding to the first housing 112 and the second housing 122 integrated together. In the example of FIGs. 10 to 14, the housing 110 includes, for example, at least a part of the flow path section 21 and surrounds the flow path 22. In other words, the through-hole 10h in the housing 110 defines at least a part of the flow path 22. The housing 110 may be, for example, in any external shape such as cubic, rectangular, or cylindrical. The housing 110 includes, for example, the through-hole 10h. The housing 110 includes, for example, the first opening Op1 and the second opening Op2. The first opening Op1 is, for example, at an end (first end) of the flow path 22 in the housing 110 in the longitudinal direction (e.g., positive Z-direction). The second opening Op2 is, for example, at the end (second end) of the flow path 22 in the housing 110 opposite to the first end in the longitudinal direction (e.g., positive Z-direction). In the example of FIG. 10 to 14, the through-hole 10h extends through the housing 110 in, for example, the positive Z-direction. The through-hole 10h includes the first opening Op1 in a portion of the housing 110 in the positive Z-direction and the second opening Op2 in a portion of the housing 110 in the negative Z-direction. The housing 110 may be a single member with the through-hole 10h or include two or more members connected together to define the through-hole 10h. The single member may be defined by two or more parts connected together.

The housing 110 includes, for example, a recess (also referred to as a fourth recess) 110r with its opening connected to the through-hole 10h in the internal space of the housing 110. In the example of FIGs. 10 to 14, the fourth recess 110r includes the opening connected to the through-hole 10h and facing in the positive X-direction. The opening (also referred to as a third opening) Op3 of the fourth recess 110r connected to the through-hole 10h is, for example, semicylindrical. The fourth recess 110r receives, for example, the sensor 111. More specifically, for example, the fourth recess 110r receives the mounting substrate 101 on which the sensor 111 is mounted. The third opening Op3 of the fourth recess 110r connected to the through-hole 10h receives, for example, the light transmitter 21t that partitions the fourth recess 110r from the through-hole 10h. The light transmitter 21t is, for example, semicylindrical. The light transmitter 21t is made of, for example, a light-transmissive material. Examples of the light-transmissive material include glass and a polymer resin. The reflector 121 having higher reflectance for irradiation light L1 than the light transmitter 21t is located, for example, along a portion of the inner surface of the through-hole 10h facing the light transmitter 21t.

In this example, the portion as the inner surface of the through-hole 10h, the light transmitter 21t, and the reflector 121 together serve as the flow path section 21 that includes, in its internal space, the flow path 22 on which the fluid 3 flows. In other words, the flow path section 21 includes, for example, the light transmitter 21t that transmits light (irradiation light L1) emitted from the light emitter 1111. The housing 110 includes, for example, an annular fitting portion (also referred to as a first fitting portion) 211 defining the first opening Op1 and an annular fitting portion (also referred to as a second fitting portion) 212 defining the second opening Op2. The housing 110 with the above structure may include, for example, two or more parts connected together.

As in the example of FIGs. 10 to 12, the first fitting portion 211 may be fitted with an end of a tubular body (also referred to as a first tubular body) 4. This connects the flow path section 21 and the first tubular body 4 together. As in the example of FIGs. 10 to 12, the second fitting portion 212 may be fitted with an end of a tubular body (also referred to as a second tubular body) 5. This connects the flow path section 21 and the second tubular body 5 together. The first tubular body 4 and the second tubular body 5 include, for example, tubular objects (tubular bodies) such as pipes in any device. The measurement module 1 according to the second embodiment is connected to pipes in any device (also referred to as being in a connected state) to be located between the pipes to quantitatively measure the state of the fluid 3 flowing through the pipes. When, for example, the fluid 3 flows from the first tubular body 4 to the second tubular body 5, the measurement module 1 in the connected state allows the fluid 3 to flow through the flow path 22 in the internal space of the flow path section 21. The gap between the first fitting portion 211 and the first tubular body 4 may be, for example, sealed with a resin gasket, and the gap between the second fitting portion 212 and the second tubular body 5 may be sealed with a resin gasket. The first fitting portion 211 and the first tubular body 4 may be firmly connected together by, for example, adhesion, crimping, or tightening. The second fitting portion 212 and the second tubular body 5 may be firmly connected together by, for example, adhesion, crimping, or tightening. The first tubular body 4 and the second tubular body 5 serving as pipes in any device may be made of, for example, a light-transmissive material or non-light-transmissive material.

In the connected state, the light emitter 1111 can, for example, irradiate the light transmitter 21t being a part of the flow path section 21 with irradiation light L1. Although a portion of the irradiation light L1 is, for example, scattered by the light transmitter 21t, another portion of the irradiation light L1 is transmitted through the light transmitter 21t and reaches the reflector 121 and the fluid 3 flowing through the flow path 22 in the internal space 21i of the flow path section 21. In the connected state, the light receiver 1112 can thus receive, in response to, for example, the light emitter 1111 irradiating the flow path section 21 with irradiation light L1, light L2a scattered by the light transmitter 21t being a part of the flow path section 21, light L2b transmitted through the light transmitter 21t and scattered by the fluid 3 on the flow path 22, and light L2c transmitted through the light transmitter 21t and the fluid 3 on the flow path 22 and reflected by the reflector 121. The presence of light L2c may increase, for example, the intensity I of the coherent light L2 received by the light receiver 1112 in response to the light emitter 1111 irradiating the flow path section 21 with irradiation light L1 and the amplitude of the optical beat signal component in the signal output from the light receiver 1112. This may thus improve, for example, the measurement accuracy of the quantitative value (flow quantitative value) indicating the flow state of the fluid 3 calculated by analysis on the optical beat signal. Thus, the accuracy of quantitative measurement of the flow state of the fluid 3 on the flow path 22 may be improved.

A housing 110 having higher light-shielding than the light transmitter 21t may, for example, obstruct the optical path of light from outside the measurement module 1 to the light receiver 1112. This can improve, for example, the measurement accuracy for the flow state of the fluid 3 on the flow path 22. In the same or similar manner as the first housing 112 and the second housing 122, the housing 110 may be made of, for example, a black resin having high light-shielding for visible light or a metal material or a ceramic material having high light-shielding for visible light.

The reflector 121 may be, for example, distant from the first opening Op1 in the through-hole 10h as in FIG. 10. In this case, light entering the through-hole 10h from outside the measurement module 1 through the first opening Op1 is less likely to reach the light receiver 1112 due to the reflection by the reflector 121. Thus, the measurement module 1 in the connected state is less likely to allow light from outside the measurement module 1 to enter the light receiver 1112 with a first tubular body 4 made of a light-transmissive material. This can improve, for example, the accuracy of quantitative measurement of the flow state of the fluid 3 on the flow path 22. The reflector 121 may be, for example, distant from the second opening Op2 in the through-hole 10h as in FIG. 10. In this case, light entering the through-hole 10h from outside the measurement module 1 through the second opening Op2 is less likely to reach the light receiver 1112 due to the reflection by the reflector 121. Thus, the measurement module 1 in the connected state is less likely to allow light from outside the measurement module 1 to enter the light receiver 1112 with a second tubular body 5 made of a light-transmissive material. This can improve, for example, the accuracy of quantitative measurement of the flow state of the fluid 3 on the flow path 22. The portion of the inner surface of the through-hole 10h excluding the reflector 121 may have, for example, reflectance for light lower than the reflectance of the reflector 121 for irradiation light L1.

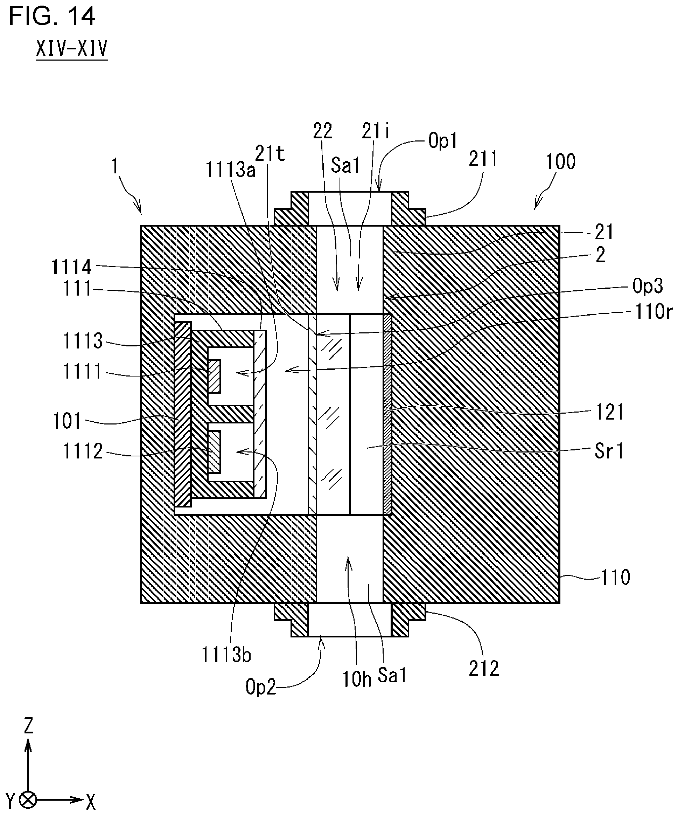

In other words, as illustrated in the example of FIG. 14, the reflector 121 includes a surface (reflective surface) Sr1 facing the sensor 111 across the flow path section 21. The portion of the inner surface of the through-hole 10h not receiving the reflector 121 may define, for example, a surface (low reflective surface) Sa1 having lower reflectance for light than the reflective surface Sr1. The low reflective surface Sa1 may be made of, for example, a black material such as a black resin. The low reflective surface Sa1 may be made of, for example, the material for the housing 110, or may be a black film extending along the inner surface of the through-hole 10h. The black film may be formed by, for example, deposition of a black resin by coating or other techniques. The black film may be attached along the inner surface of the through-hole 10h with, for example, an adhesive or adhesive tape.

2-2. Third Embodiment

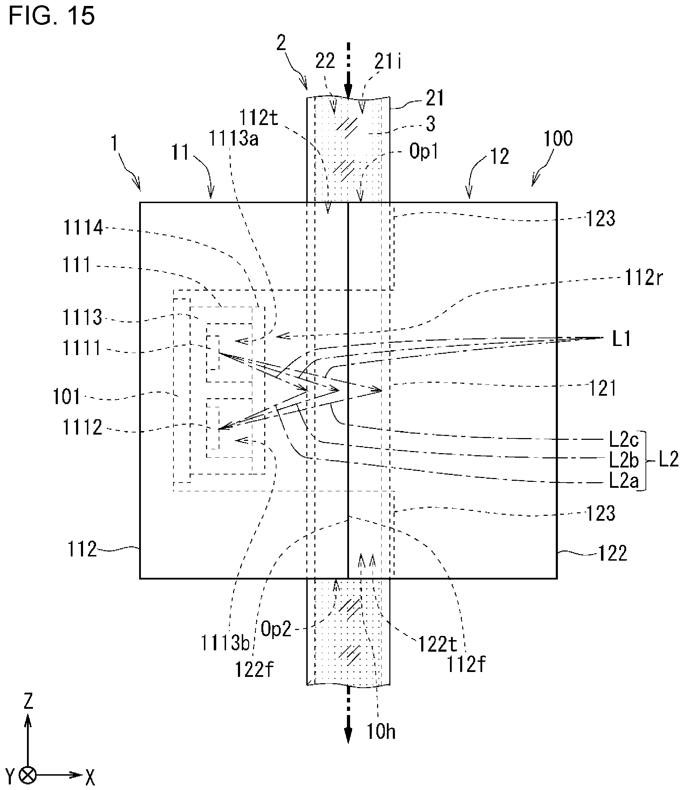

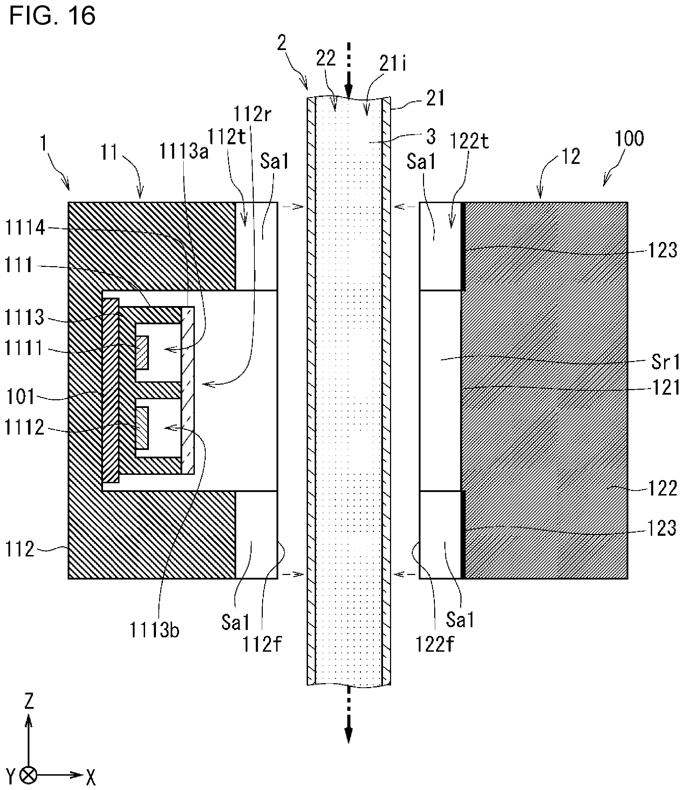

In the first embodiment, for example, the reflector 121 may be included in the second housing 122, as illustrated in FIGs. 15 and 16. The second housing 122 may be made of, for example, a material having higher reflectance for irradiation light L1 than the material for the flow path section 21. For example, the second housing 122 may be made of a metal such as aluminum or copper. In a third embodiment illustrated in FIGs. 15 and 16, a measurement module 1 with an example structure includes a reflector 121 serving as the bottom of the second groove 122t on the second housing 122. The bottom of the second groove 122t may include, for example, portions (also referred to as low reflective portions) 123 serving as low reflective surfaces Sa1 with lower light reflectance than the reflective surface Sr1 along the two end portions of the second groove 122t in the longitudinal direction (e.g., positive Z-direction) across the reflector 121. Each low reflective portion 123 may be a black film made of, for example, a black resin along the bottom of the second groove 122t. The black film may be formed by, for example, deposition by coating or other techniques. The black film may be attached along the bottom of the second groove 122t with, for example, an adhesive or adhesive tape. At least a portion of the bottom (inner surface) of the second groove 122t on the second housing 122 may have, for example, a lower surface roughness to increase the light reflectance of the reflective surface Sr1 included in the reflector 121. The two end portions of the bottom (inner surface) of the second groove 122t on the second housing 122 in the longitudinal direction (e.g., positive Z-direction) of the second groove 122t across the reflective surface Sr1 may have a higher surface roughness than the reflective surface Sr1 to provide the low reflective portions 123 including the low reflective surfaces Sa1 with lower light reflectance than the reflective surface Sr1. In other words, the second housing 122 may have a difference in surface roughness or material to provide the reflector 121 and the low reflective portions 123 having different surface states.

### 2-3. Fourth Embodiment

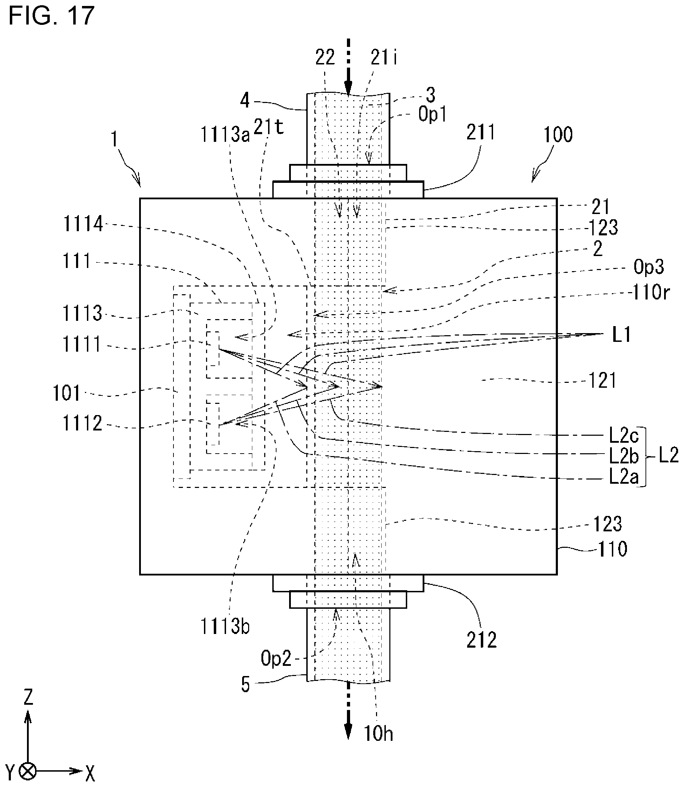

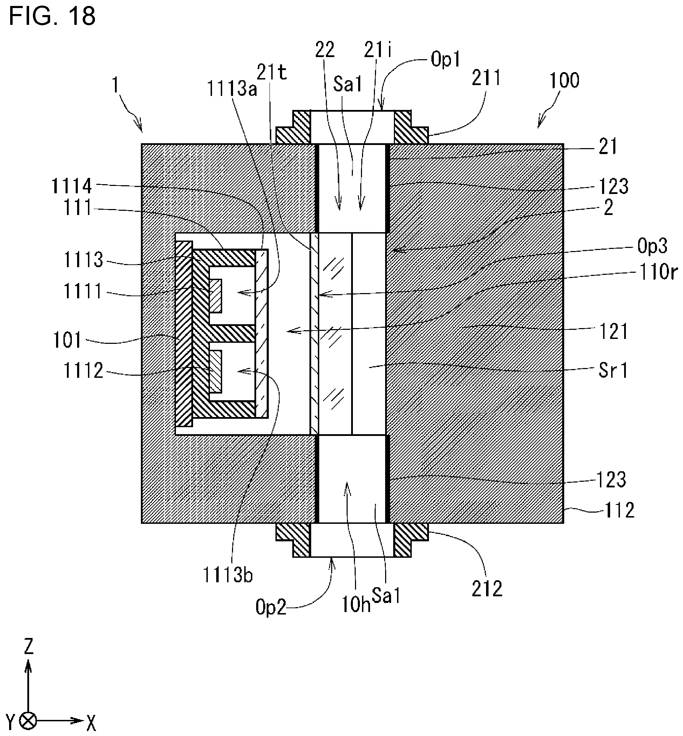

In the second embodiment, for example, the reflector 121 may be included in the housing 110, as illustrated in FIGs. 17 and 18. The housing 110 may be made of, for example, a material having higher reflectance for irradiation light L1 than the material for the flow path section 21 such as the light transmitter 21t. For example, the housing 110 may be made of a metal such as aluminum or copper. In a fourth embodiment illustrated in FIGs. 17 and 18, a measurement module 1 with an example structure includes a reflector 121 serving as the inner surface of the through-hole 10h in the housing 110. The inner surface of the through-hole 10h in the housing 110 may include, for example, portions (low reflective portions) 123 serving as the low reflective surfaces Sa1 with lower light reflectance than the reflective surface Sr1 along the two end portions of the through-hole 10h across the reflector 121 in the longitudinal direction (e.g., positive Z-direction) of the through-hole 10h. In other words, for example, one of the low reflective portions 123 may be located in a portion of the inner surface of the through-hole 10h closer to the first opening Op1 than the reflector 121, and another of the low reflective portions 123 may be located in a portion of the inner surface of the through-hole 10h closer to the second opening Op2 than the reflector 121. Each low reflective portion 123 may be, for example, a circular tube extending along the inner surface of the through-hole 10h. Each low reflective portion 123 may be a black film made of, for example, a black resin along the inner surface of the through-hole 10h. The black film may be formed by, for example, deposition by coating or other techniques. The black film may be attached along the inner surface of the through-hole 10h with, for example, an adhesive or adhesive tape.

### 2-4. Fifth Embodiment

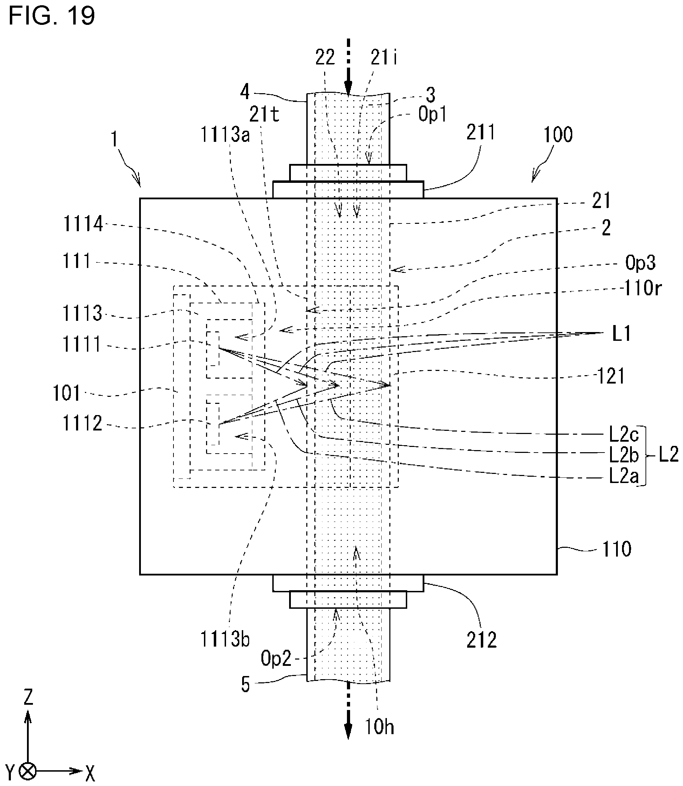

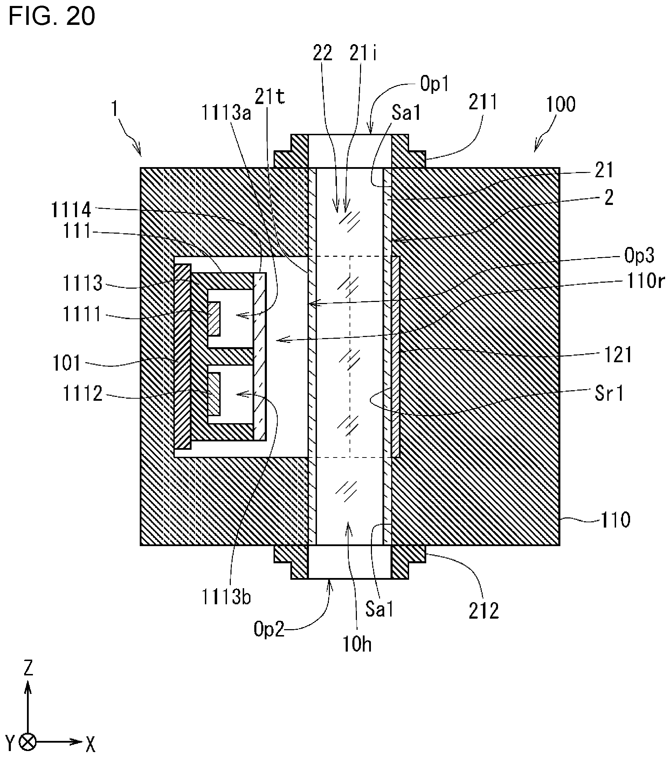

In each of the second and fourth embodiments, for example, the measurement module 1 may include the flow path section 21 located in the through-hole 10h in the housing 110, as illustrated in FIGs. 19 and 20. In other words, for example, the housing 110 may surround the flow path section 21. The flow path section 21 may include, for example, a tubular body. In this case, for example, the light transmitter 21t may define at least a part of the flow path section 21. The housing 110 may have, for example, higher light-shielding than the flow path section 21. In this case, light from outside the measurement module 1 is less likely to enter the light receiver 1112. This can improve, for example, the accuracy of quantitative measurement of the flow state of the fluid 3 on the flow path 22.
In a fifth embodiment illustrated in FIGs. 19 and 20, a measurement module 1 with an example structure includes a light transmitter 21t defining a portion of the flow path section 21 at least along the third opening Op3. The light emitter 1111 can thus irradiate, for example, the light transmitter 21t being a part of the flow path section 21 with irradiation light L1 in the connected state. The flow path section 21 may be made of, for example, a light-transmissive material. Examples of the light-transmissive material include glass and a polymer resin. A flow path section 21 made of a light-transmissive material may be a single member in the measurement module 1. The inner surface of the flow path section 21 is, for example, thus less likely to be staggered between different members. The inner surface of the flow path section 21 is also less likely to have surface roughness differences between different members. Thus, the flow of the fluid 3 is less likely to be obstructed in the flow path 22 in the internal space of the flow path section 21. This can improve, for example, the accuracy of quantitative measurement of the flow state of the fluid 3 on the flow path 22.
The reflector 121 faces the sensor 111 across, for example, at least a part of the flow path 22. With this structure, although a portion of irradiation light L1 emitted from the light emitter 1111 onto the light transmitter 21t being a part of the flow path section 21 is, for example, scattered by the light transmitter 21t in the connected state, another portion of the irradiation light L1 is transmitted through the light transmitter 21t and reaches, for example, the reflector 121 and the fluid 3 flowing through the flow path 22 in the internal space 21i of the flow path section 21.
The reflector 121 may be, for example, located on a portion of the outer surface of the flow path section 21 opposite to the flow path 22 or located outside the flow path section 21. In this case, the reflector 121 is unexposed to the flow path 22. The material of the reflector 121 may thus be less likely to dissolve into the fluid 3 flowing through the flow path 22 and be less likely to corrode or stain the reflector 121. This can improve, for example, the accuracy of quantitative measurement of the flow state of the fluid 3 on the flow path 22.

A reflector 121 located on, for example, a portion of the outer surface of the flow path section 21 opposite to the flow path 22 can increase the intensity of light L2c transmitted through the light transmitter 21t being a part of the flow path section 21 and through the fluid 3 on the flow path 22 and reflected by the reflector 121. This may increase, for example, the intensity I of the coherent light L2 received by the light receiver 1112 in response to the light emitter 1111 irradiating the flow path section 21 with irradiation light L1 and the amplitude of the optical beat signal component in the signal output from the light receiver 1112. This may thus improve, for example, the measurement accuracy of the quantitative value (flow quantitative value) indicating the flow state of the fluid 3 calculated by analysis on the optical beat signal. Thus, the accuracy of quantitative measurement of the flow state of the fluid 3 on the flow path 22 may be improved.

A film- or foil-like reflector 121 may be, for example, located on the outer surface of a tubular body serving as the flow path section 21 to provide the reflector 121 in a portion of the outer surface of the flow path section 21 opposite to the flow path 22. The film- or foil-like reflector 121 may be, for example, formed on the outer surface of the tubular body serving as the flow path section 21 by dry deposition such as vapor deposition or sputtering or by wet deposition such as plating or coating. A film-, foil-, or plate-like reflector 121 may be located on the outer surface of the tubular body serving as the flow path section 21 by bonding with, for example, an adhesive or attachment with, for example, adhesive tape.

The reflector 121 may be located along, for example, a portion of the inner surface of through-hole 10h in the housing 110 facing the light transmitter 21t to be outside the flow path section 21. A film- or foil-like reflector 121 may be, for example, formed in a portion of the inner surface of the through-hole 10h in the housing 110 by dry deposition such as vapor deposition or sputtering or by wet deposition such as plating or coating. A film-, foil-, or plate-like reflector 121 may be located on a portion of the inner surface of the through-hole 10h in the housing 110 by bonding with, for example, an adhesive or attachment with, for example, adhesive tape.

A film- or foil-like reflector 121 may be, for example, located along the inner surface of the flow path section 21.

2-5. Sixth Embodiment

In each of the above first and third embodiments, the measurement module 1 may include, for example, an optical filter 15 to be located between the flow path section 21 and the light receiver 1112 in the first state. The optical filter 15 allows, for example, transmission of light in a predetermined wavelength range (also referred to as a first wavelength range) including the wavelength of irradiation light L1 emitted from the light emitter 1111 and allows transmission of less light in a predetermined wavelength range (also referred to as a second wavelength range) different from the first wavelength range. The optical filter 15 is, for example, a bandpass filter with a dielectric multilayer film. For example, the first wavelength range may include a wavelength of about 850 nm, which is the wavelength of irradiation light L1. The second wavelength range may include a wavelength range of 400 to 700 nm, which is the wavelength range of visible light. This structure including, for example, the optical filter 15 is less likely to allow light from outside the measurement module 1 to enter the light receiver 1112. This can improve, for example, the accuracy of quantitative measurement of the flow state of the fluid 3 on the flow path 22.

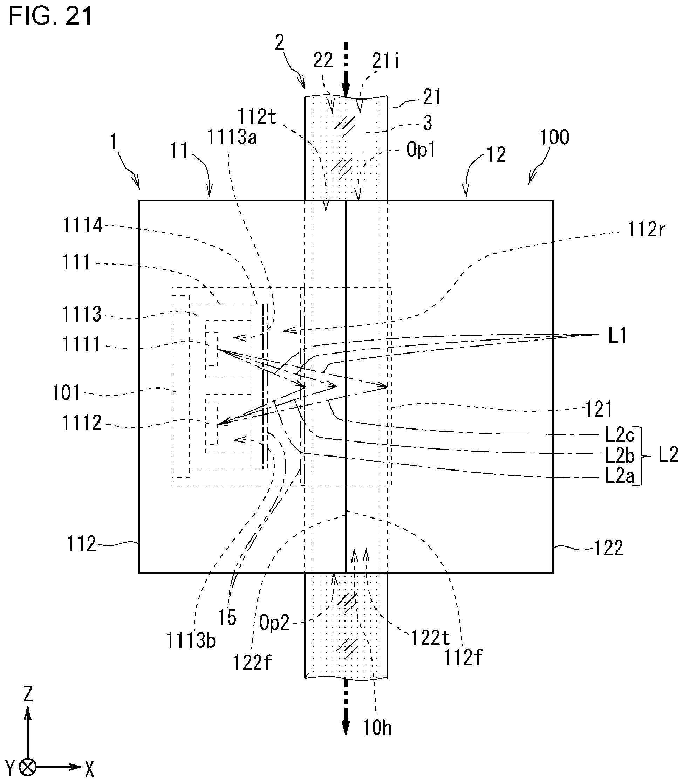

A measurement module 1 with an example structure according to a sixth embodiment illustrated in FIG. 21 is based on the structure of the measurement module 1 according to the first embodiment illustrated in FIG. 1. The measurement module 1 with the structure according to the sixth embodiment additionally includes an optical filter 15 to be located between the flow path section 21 and the light receiver 1112 in the first state. The optical filter 15 may be attached to, for example, the sensor 111 or the flow path section 21. More specifically, the optical filter 15 may be located on, for example, the lid 1114 or the surface of the flow path section 21.

2-6. Seventh Embodiment

In each of the above second, fourth, and fifth embodiments, the measurement module 1 may include, for example, an optical filter 15 between the flow path 22 and the light receiver 1112. The optical filter 15 allows, for example, transmission of light in the first wavelength range including the wavelength of irradiation light L1 emitted from the light emitter 1111 and allows transmission of less light in the second wavelength range different from the first wavelength range. The optical filter 15 is, for example, a bandpass filter with a dielectric multilayer film. The first wavelength range may include, for example, a wavelength of about 850 nm, which is the wavelength of irradiation light L1. The second wavelength range may include a wavelength range of 400 to 700 nm, which is the wavelength range of visible light. This structure including, for example, the optical filter 15 is less likely to allow light from outside the measurement module 1 to enter the light receiver 1112. This can improve, for example, the accuracy of quantitative measurement of the flow state of the fluid 3 on the flow path 22.

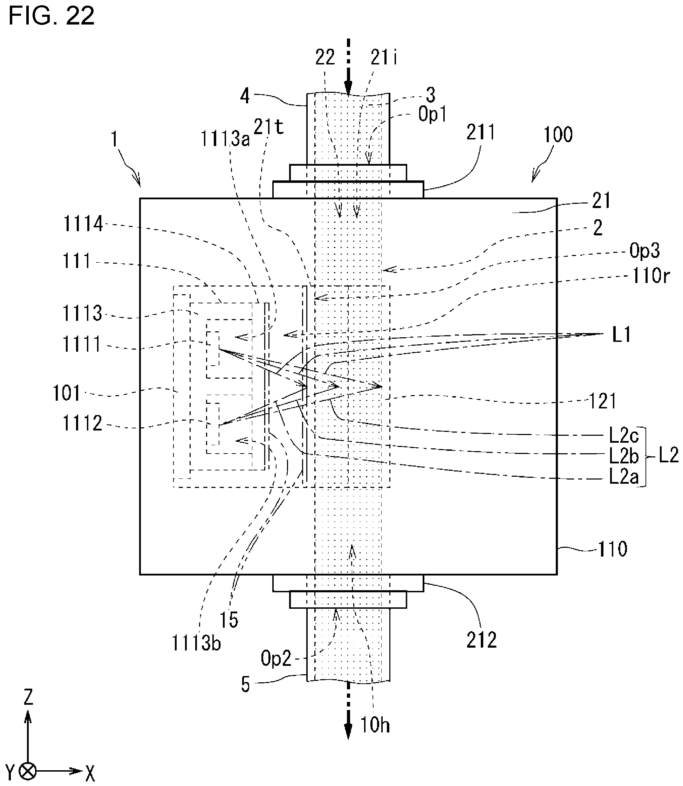

A measurement module 1 with a first structure according to a seventh embodiment illustrated in FIG. 22 is based on the structure of the measurement module 1 according to the second embodiment illustrated in FIG. 10. The measurement module 1 with the first structure according to the seventh embodiment additionally includes an optical filter 15 between the flow path 22 and the light receiver 1112. In this example, the optical filter 15 may be located on the lid 1114 or the surface of the light transmitter 21t included in the flow path section 21.

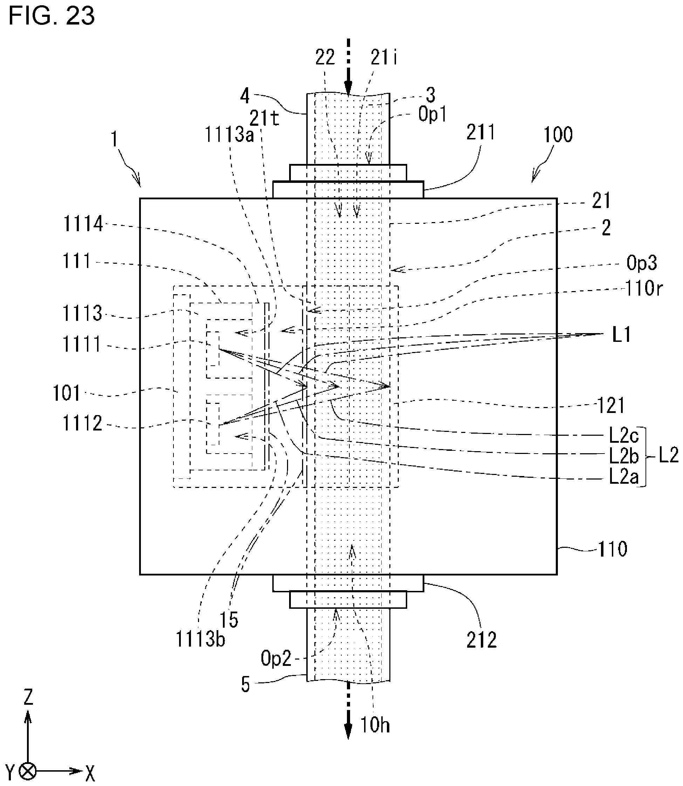

A measurement module 1 with a second structure according to the seventh embodiment illustrated in FIG. 23 is based on the structure of the measurement module 1 according to the fourth embodiment illustrated in FIG. 19. The measurement module 1 with the second structure according to the seventh embodiment additionally includes an optical filter 15 between the flow path 22 and the light receiver 1112. In this example as well, the optical filter 15 may be located on the lid 1114 or the surface of the light transmitter 21t included in the flow path section 21.

2-7. Eighth Embodiment

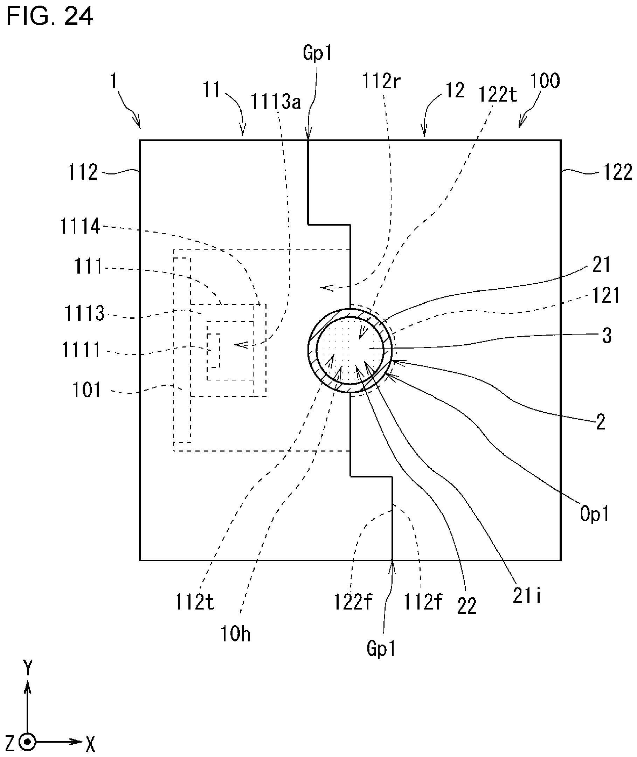

In each of the above first, third, and sixth embodiments, as illustrated in, for example, FIGs. 24 to 35, the first surface 112f of the first housing 112 and the second surface 122f of the second housing 122 may be bent in a direction away from an area in which the flow path section 21 is located in the third state. This structure allows, for example, less light from outside the measurement module 1 to reach, through any gap Gp1 left between the first surface 112f and the second surface 122f in the third state, the through-hole 10h defining the flow path section 21 in the third state. This allows, for example, less light to enter the light receiver 1112 from outside the measurement module 1 in quantitative measurement of the flow state of the fluid 3 on the flow path 22 defined in the internal space 21i of the flow path section 21. This can improve, for example, the accuracy of quantitative measurement of the flow state of the fluid 3 on the flow path 22.

A measurement module 1 with a first structure according to an eighth embodiment illustrated in FIG. 24 is based on the structure of the measurement module 1 according to the first embodiment illustrated in FIG. 2. In the measurement module 1 with the first structure according to the eighth embodiment, each of the first surface 112f of the first housing 112 and the second surface 122f of the second housing 122 is bent in a direction away from the through-hole 10h in the third state.

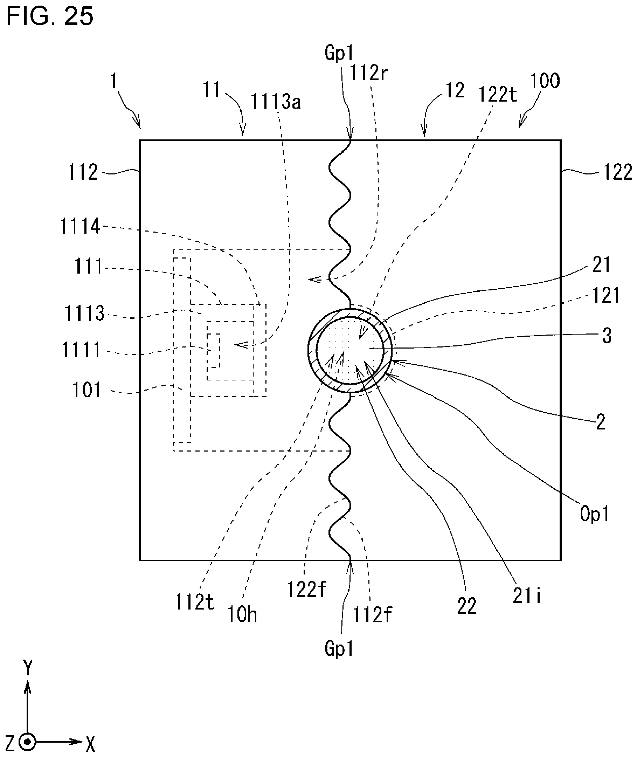

A measurement module 1 with a second structure according to the eighth embodiment illustrated in FIG. 25 is based on the structure of the measurement module 1 according to the first embodiment illustrated in FIG. 2. In the measurement module 1 with the second structure according to the eighth embodiment, each of the first surface 112f of the first housing 112 and the second surface 122f of the second housing 122 is curved in a direction away from the through-hole 10h in the third state. As in the example of FIG. 25, the first surface 112f and the second surface 122f may each include several bends to allow less light to enter the light receiver 1112 from outside the measurement module 1.

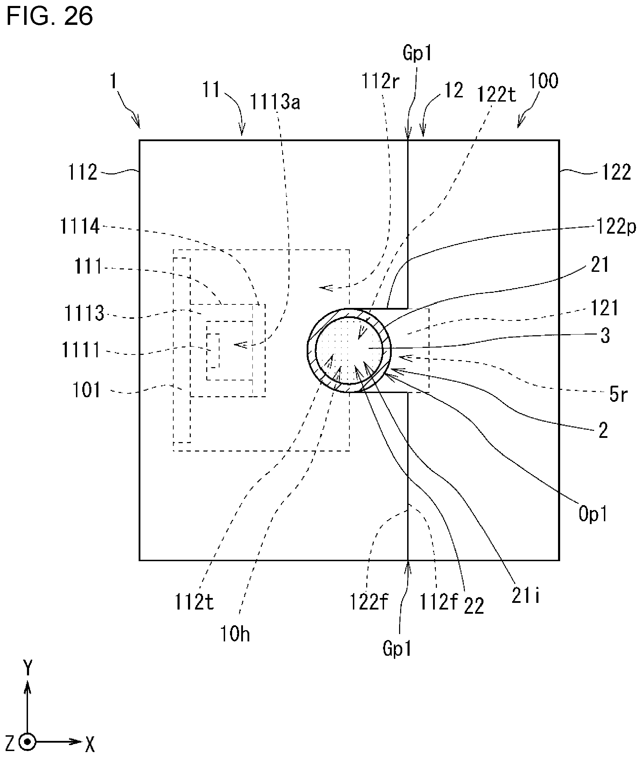

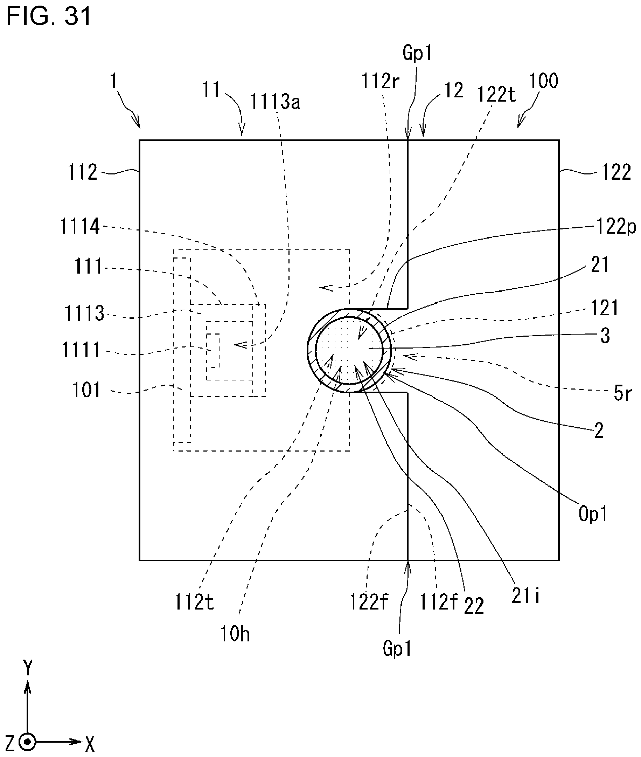

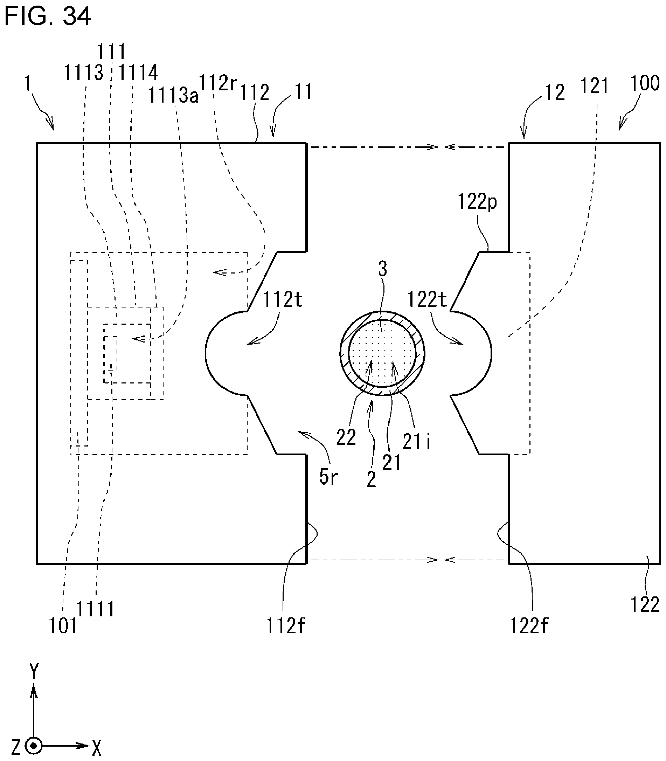

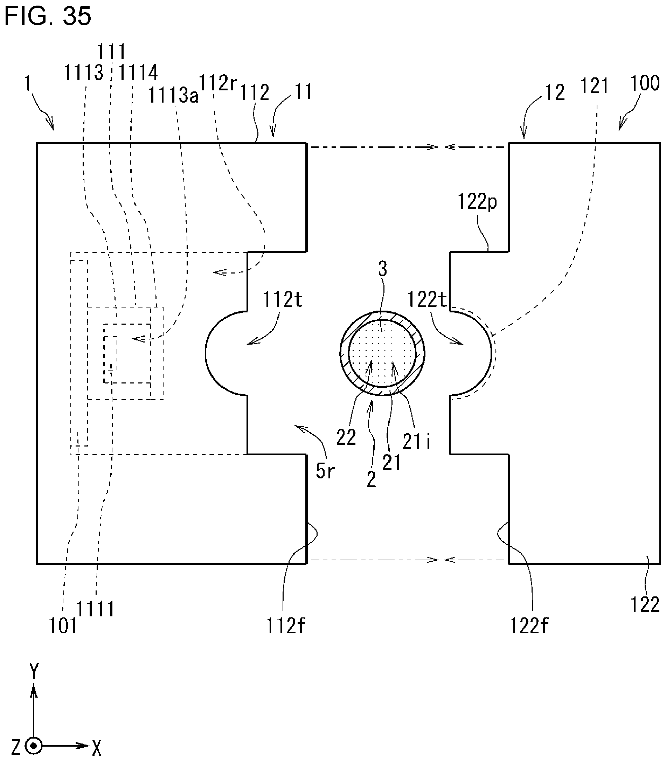

A measurement module 1 with a third structure according to the eighth embodiment illustrated in FIGs. 26 to 30 is based on the structure of the measurement module 1 according to the first embodiment illustrated in FIGs. 1 to 5. More specifically, the first housing 112 is enlarged and includes a recess (also referred to as a fifth recess) 5r with its innermost portion (bottom) defined by the first groove 112t. The second housing 122 is reduced in size and additionally includes a protrusion 122p extending from the second surface 122f of the second housing 122 and including the second groove 122t at its tip. The fifth recess 5r is located on, for example, the first surface 112f of the first housing 112. The fifth recess 5r extends, for example, along the first groove 112t. In the example of FIGs. 26 to 30, the fifth recess 5r is, for example, recessed in the negative X-direction and extends in Z-direction along the first groove 112t. The protrusion 122p has, for example, a shape to fit into the fifth recess 5r to allow the first groove 112t and the second groove 122t to define the through-hole 10h in the third state. The first housing 112 thus includes, for example, a portion protruding more in the positive X-direction than the flow path section 21 in the third state. In the example of FIGs. 26 to 30, the fifth recess 5r includes an internal space defined by a prismatic internal space recessed in the negative X-direction and a semi-prismatic internal space within the first groove 112t connected together. The protrusion 122p includes the second groove 122t in a portion of the prismatic portion in the negative X-direction. As illustrated in the example of FIG. 26, in the third state, the first surface 112f includes a surface extending in the positive X-direction from a portion of the first surface 112f inside the through-hole 10h receiving the flow path section 21 and bending in the positive Y-direction and a surface extending in the positive X-direction from a portion of the first surface 112f inside the through-hole 10h receiving the flow path section 21 and bending in the negative Y-direction. As illustrated in the example of FIG. 26, in the third state, the second surface 122f includes a surface extending in the positive X-direction from a portion of the second surface 122f inside the through-hole 10h receiving the flow path section 21 and bending in the positive Y-direction and a surface extending in the positive X-direction from a portion of the second surface 122f inside the through-hole 10h receiving the flow path section 21 and bending in the negative Y-direction. The reflector 121 is located along, for example, a portion of the bottom (inner surface) of the second groove 122t in Z-direction. The reflector 121 may have, for example, any structure to be located along a portion of the bottom (inner surface) of the second groove 122t in Z-direction. For example, as illustrated in the example of FIGs. 26 to 30, the reflector 121 may define a part of a section of the protrusion 122p in Z-direction and may be embedded in the second housing 122. For example, as in a measurement module 1 with a fourth structure according to the eighth embodiment illustrated in FIGs. 31 to 33, the reflector 121 may be semicylindrical and located along a portion of the bottom (inner surface) of the second groove 122t in Z-direction. The recess 5r may have, for example, any shape to receive the protrusion 122p. For example, as illustrated in FIGs. 34 and 35, the fifth recess 5r may have a greater width than the first groove 112t in the direction orthogonal to the longitudinal direction of the first groove 112t. In this case, the protrusion 122p may have a greater width than the second groove 122t in the direction orthogonal to the longitudinal direction of the second groove 122t. A measurement module 1 with a fifth structure according to the eighth embodiment illustrated in FIG. 34 is based on the third structure of the measurement module 1 according to the eighth embodiment illustrated in FIGs. 26 to 30. In the measurement module 1 with the fifth structure according to the eighth embodiment, the fifth recess 5r has a greater width than the first groove 112t, and the protrusion 122p has a greater width than the second groove 122t in Y-direction. A measurement module 1 with a sixth structure according to the eighth embodiment illustrated in FIG. 35 is based on the fourth structure of the measurement module 1 according to the eighth embodiment illustrated in FIGs. 31 to 33. In the measurement module 1 with the sixth structure according to the eighth embodiment, the fifth recess 5r has a greater width than the first groove 112t, and the protrusion 122p has a greater width than the second groove 122t in Y-direction.

2-8. Ninth Embodiment

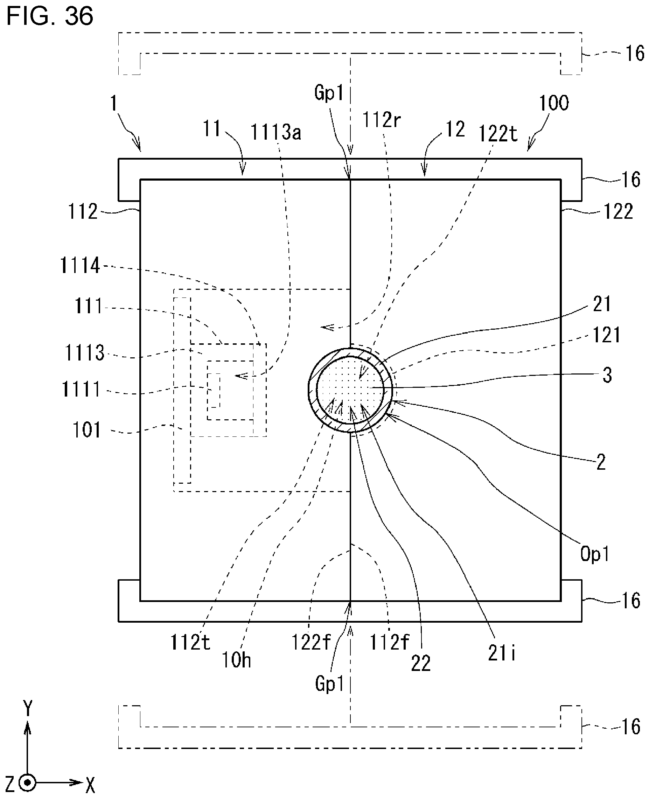

In each of the above first, third, sixth, and eighth embodiments, as illustrated in FIGs. 36 to 40, the measurement module 1 may include, for example, covers 16 to be located to cover a gap Gp1 between the first surface 112f of the first housing 112 and the second surface 122f of the second housing 122 in the third state. This structure allows, for example, less light from outside the measurement module 1 to reach, through any gap Gp1 left between the first surface 112f and the second surface 122f in the third state, the through-hole 10h defining the flow path section 21 in the third state. This allows, for example, less light to enter the light receiver 1112 from outside the measurement module 1 in quantitative measurement of the flow state of the fluid 3 on the flow path 22 defined in the internal space 21i of the flow path section 21. This can improve, for example, the accuracy of quantitative measurement of the flow state of the fluid 3 on the flow path 22.

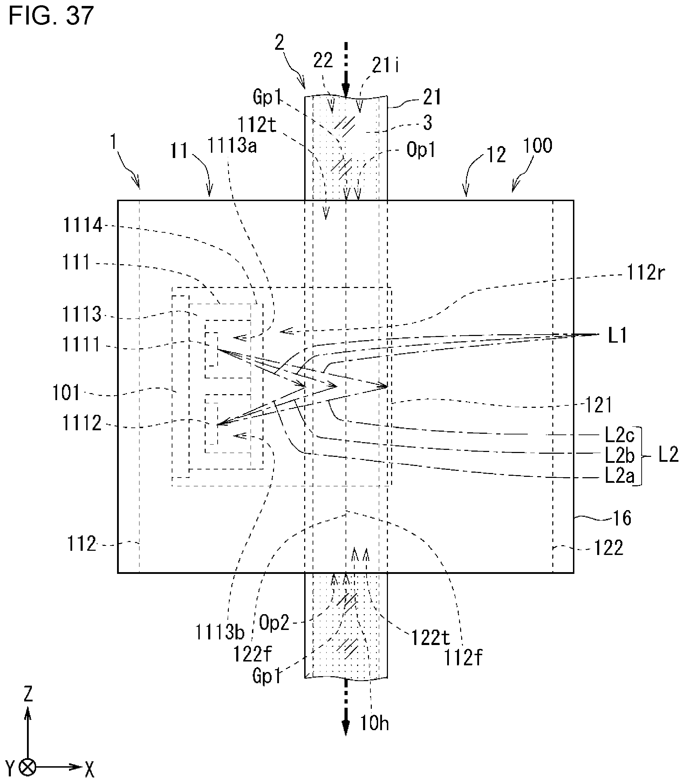

A measurement module 1 with a first structure according to a ninth embodiment illustrated in FIGs. 36 and 37 is based on the structure of the measurement module 1 according to the first embodiment illustrated in FIGs. 1 and 2. The measurement module 1 with the first structure according to the ninth embodiment additionally includes a cover 16 extending, in the third state, over the surface of the first housing 112 in the positive Y-direction and the surface of the second housing 122 in the positive Y-direction and a cover 16 extending, in the third state, over the surface of the first housing 112 in the negative Y-direction and the surface of the second housing 122 in the negative Y-direction. The covers 16 may be, for example, fixed to the first housing 112 and the second housing 122 by fitting, fastening, or bonding.

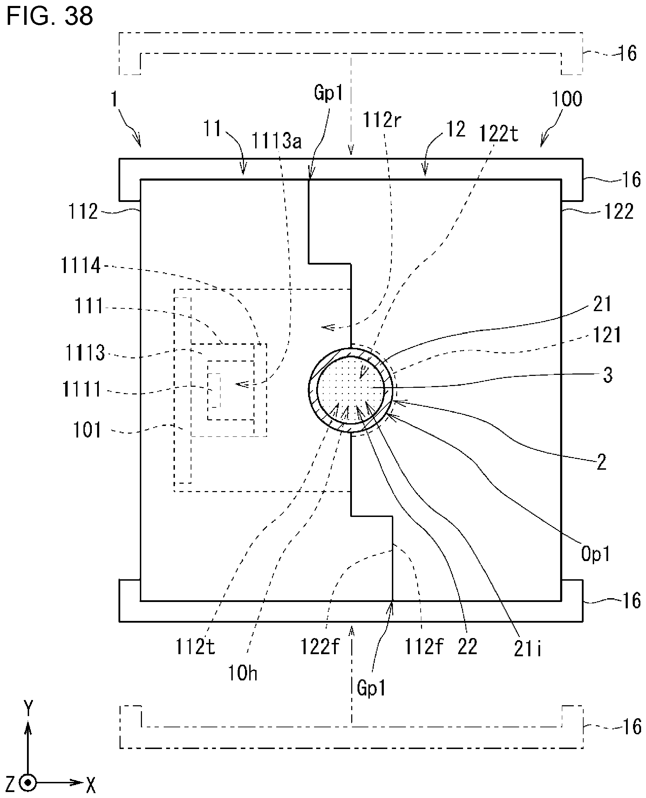

A measurement module 1 with a second structure according to the ninth embodiment illustrated in FIG. 38 is based on the first structure of the measurement module 1 according to the eighth embodiment illustrated in FIG. 24. The measurement module 1 with the second structure according to the ninth embodiment additionally includes a cover 16 extending, in the third state, over the surface of the first housing 112 in the positive Y-direction and the surface of the second housing 122 in the positive Y-direction and a cover 16 extending, in the third state, over of the surface of the first housing 112 in the negative Y-direction and the surface of the second housing 122 in the negative Y-direction. The covers 16 may be, for example, fixed to the first housing 112 and the second housing 122 by fitting, fastening, or bonding.

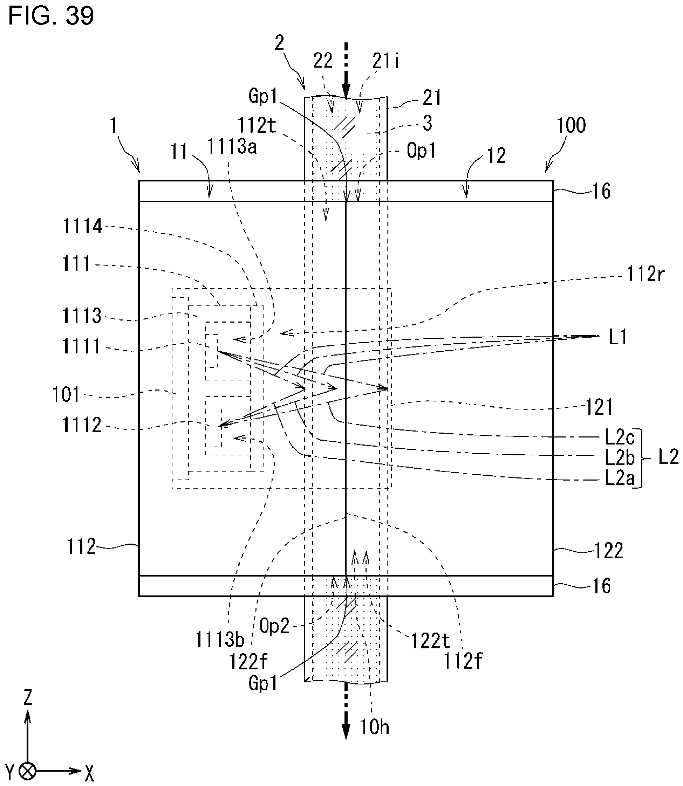

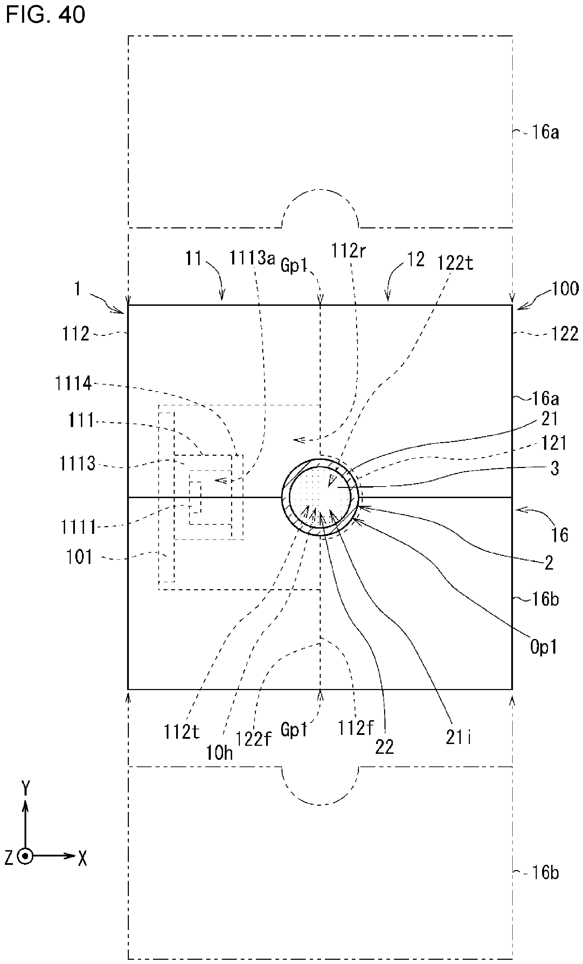

A measurement module 1 with a third structure according to the ninth embodiment illustrated in FIGs. 39 and 40 is based on the structure of the measurement module 1 according to the first embodiment illustrated in FIGs. 1 and 2. The measurement module 1 with the third structure according to the ninth embodiment additionally includes a cover 16 extending, in the third state, over the surface of the first housing 112 in the positive Z-direction and the surface of the second housing 122 in the positive Z-direction and a cover 16 extending, in the third state, over of the surface of the first housing 112 in the negative Z-direction and the surface of the second housing 122 in the negative Z-direction. As illustrated in FIG. 40, each cover 16 may include, for example, a first cover portion 16a and a second cover portion 16b that hold the flow path section 21 between them in the third state.

The measurement module 1 in this example may include at least one or two to four of the cover 16 extending, in the third state, over the surface of the first housing 112 in the positive Y-direction and the surface of the second housing 122 in the positive Y-direction, the cover 16 extending, in the third state, over the surface of the first housing 112 in the negative Y-direction and the surface of the second housing 122 in the negative Y-direction, the cover 16 extending, in the third state, over the surface of the first housing 112 in the positive Z-direction and the surface of the second housing 122 in the positive Z-direction, or a cover 16 extending, in the third state, over the surface of the first housing 112 in the negative Z-direction and the surface of the second housing 122 in the negative Z-direction.

The covers 16 may be, for example, sized and shaped in any manner to cover at least a part of the gap Gp 1 left between the first housing 112 and the second housing 122 in the third state. The covers 16 may be, for example, a resin, a filler material such as putty, or adhesive tape that has relatively high light-shielding.

2-9. Tenth Embodiment

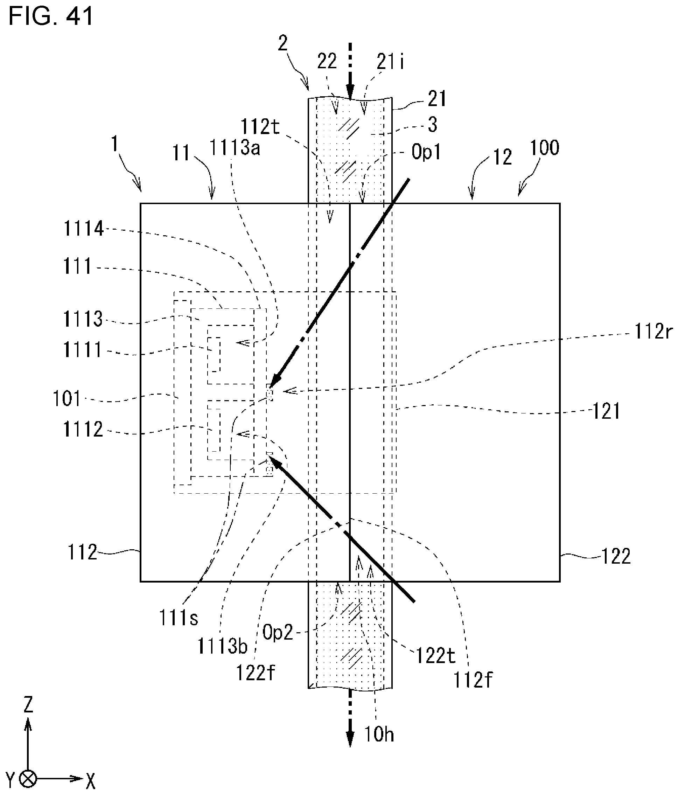

In each of the above embodiments, as illustrated in FIGs. 41 to 44, the sensor 111 may include, for example, light shields Ills that obstruct the optical path of light traveling from outside the measurement module 1 through at least one of the first opening Op1 or the second opening Op2 toward the light receiver 1112. This allows, for example, less light to enter the light receiver 1112 from outside the measurement module 1 in quantitative measurement of the flow state of the fluid 3 on the flow path 22 defined in the internal space 21i of the flow path section 21. This can improve, for example, the accuracy of quantitative measurement of the flow state of the fluid 3 on the flow path 22.

A measurement module 1 with a first structure according to a tenth embodiment illustrated in FIG. 41 is based on the structure of the measurement module 1 according to the first embodiment illustrated in FIG. 1. The measurement module 1 with the first structure according to the tenth embodiment additionally includes light shields Ills each obstructing the optical path of light traveling from outside the measurement module 1 through the first opening Op1 or the second opening Op2 toward the light receiver 1112 in the third state. In the example of FIG. 41, the light shields Ills are located on a surface of the lid 1114. In FIG. 41, the thick dot-dash lines indicate the optical paths of light traveling from outside the measurement module 1 through the first opening Op1 and the second opening Op2 toward the light receiver 1112. The measurement module 1 in this example may include the light shield Ills that obstructs, in the third state, the optical path of light traveling from outside the measurement module 1 through at least one of the first opening Op1 or the second opening Op2 toward the light receiver 1112.

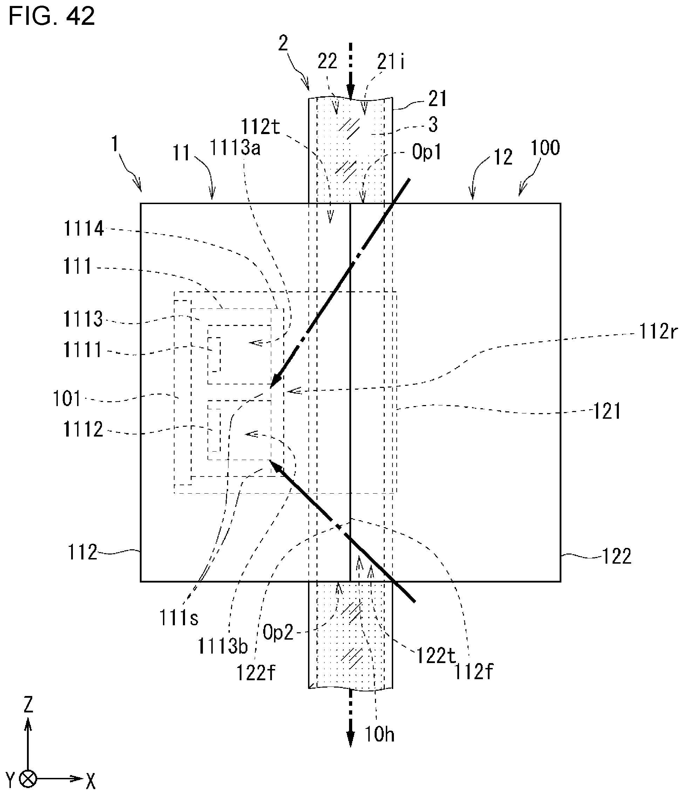

A measurement module 1 with a second structure according to the tenth embodiment illustrated in FIG. 42 is based on the structure of the measurement module 1 according to the first embodiment illustrated in FIG. 1, with the first recess 1113a and the second recess 1113b of the package 1113 having a greater depth. In this example, portions of the package 1113 serve as the light shields Ills each obstructing the optical path of light traveling from outside the measurement module 1 through the first opening Op1 or the second opening Op2 toward the light receiver 1112 in the third state. In FIG. 42 as well, the thick dot-dash lines indicate the optical paths of light traveling from outside the measurement module 1 through the first opening Op1 and the second opening Op2 toward the light receiver 1112. In this example as well, the measurement module 1 may include the light shield Ills that obstructs, in the third state, the optical path of light traveling from outside the measurement module 1 through at least one of the first opening Op1 or the second opening Op2 toward the light receiver 1112.

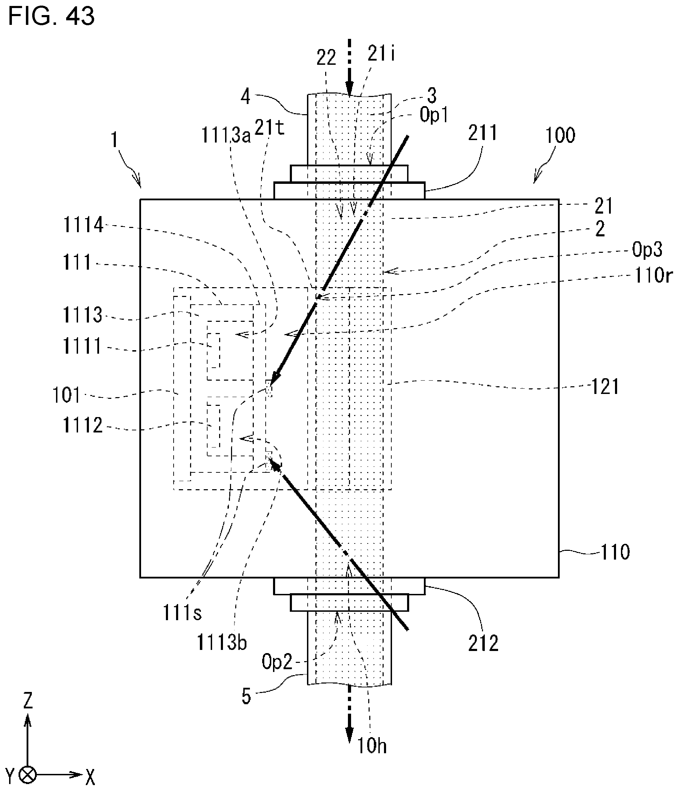

A measurement module 1 with a third structure according to the tenth embodiment illustrated in FIG. 43 is based on the structure of the measurement module 1 according to the second embodiment illustrated in FIG. 10. The measurement module 1 with the third structure according to the tenth embodiment additionally includes light shields Ills each obstructing the optical path of light traveling from outside the measurement module 1 through the first opening Op1 or the second opening Op2 toward the light receiver 1112. In the example of FIG. 43, the light shields Ills are located on a surface of the lid 1114. In FIG. 43, the thick dot-dash lines indicate the optical paths of light traveling from outside the measurement module 1 through the first opening Op1 and the second opening Op2 toward the light receiver 1112. The measurement module 1 in this example may include the light shield Ills that obstructs the optical path of light traveling from outside the measurement module 1 through at least one of the first opening Op1 or the second opening Op2 toward the light receiver 1112.

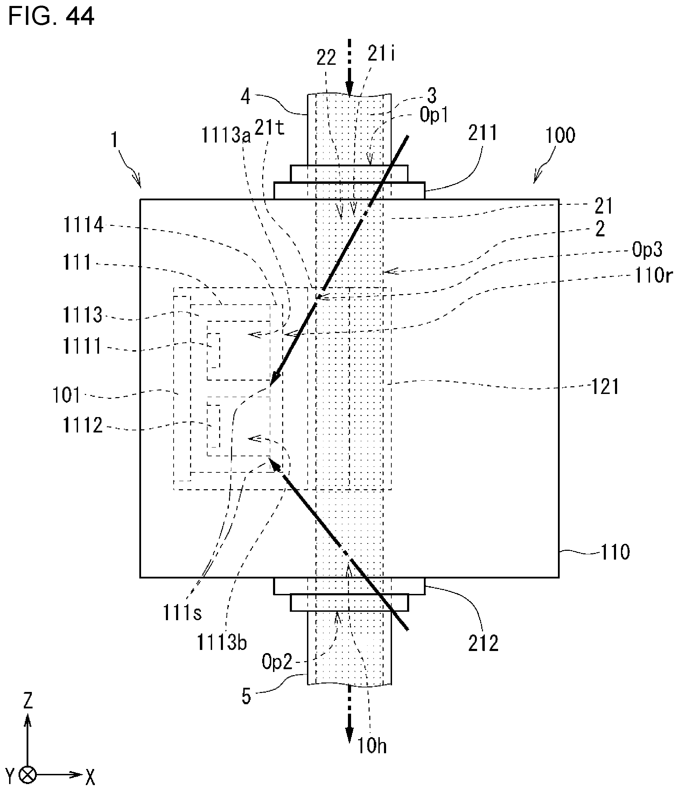

A measurement module 1 with a fourth structure according to the tenth embodiment illustrated in FIG. 44 is based on the structure of the measurement module 1 according to the second embodiment illustrated in FIG. 10. In the measurement module 1 with the fourth structure according to the tenth embodiment, the first recess 1113a and the second recess 1113b of the package 1113 each have a greater depth. In this example, portions of the package 1113 serve as the light shields Ills each obstructing the optical path of light traveling from outside the measurement module 1 through the first opening Op1 or the second opening Op2 toward the light receiver 1112. In FIG. 44 as well, the thick dot-dash lines indicate the optical paths of light traveling from outside the measurement module 1 through the first opening Op1 and the second opening Op2 toward the light receiver 1112. In this example as well, the measurement module 1 may include the light shield Ills that obstructs the optical path of light traveling from outside the measurement module 1 through at least one of the first opening Op1 or the second opening Op2 toward the light receiver 1112.

In the example of FIG. 41, the light shields Ills have higher light-shielding than, for example, the flow path section 21 and the lid 1114. In the example of FIG. 43, the light shields Ills have higher light-shielding than, for example, the light transmitter 21t and the lid 1114. The light shields 111s may include, for example, black films or films in other colors. The black films or films in other colors may be, for example, formed by dry deposition such as vapor deposition or sputtering or by wet deposition such as coating. The black films or films in other colors may be, for example, bonded with an adhesive or attached with adhesive tape to the surface of the lid 1114. The first and second housings 112 and 122 or the housing 110 are, for example, sized or shaped as appropriate. The light shields Ills are also positioned or sized as appropriate. The light shields Ills can thus obstruct the optical path of light traveling from outside the measurement module 1 through at least one the first opening Op1 or the second opening Op2 toward the light receiver 1112.

In the example of FIG. 42, the light shields Ills have higher light-shielding than, for example, the flow path section 21 and the lid 1114. In the example of FIG. 44, the light shields Ills have higher light-shielding than, for example, the light transmitter 21t and the lid 1114. The first and second housings 112 and 122 or the housing 110 are, for example, sized or shaped as appropriate. The second recess 1113b of the package 1113 has the depth and the size of its opening determined as appropriate. The package 1113 can thus obstruct the optical path of light traveling from outside the measurement module 1 through at least one the first opening Op1 or the second opening Op2 toward the light receiver 1112.

2-10. Eleventh Embodiment

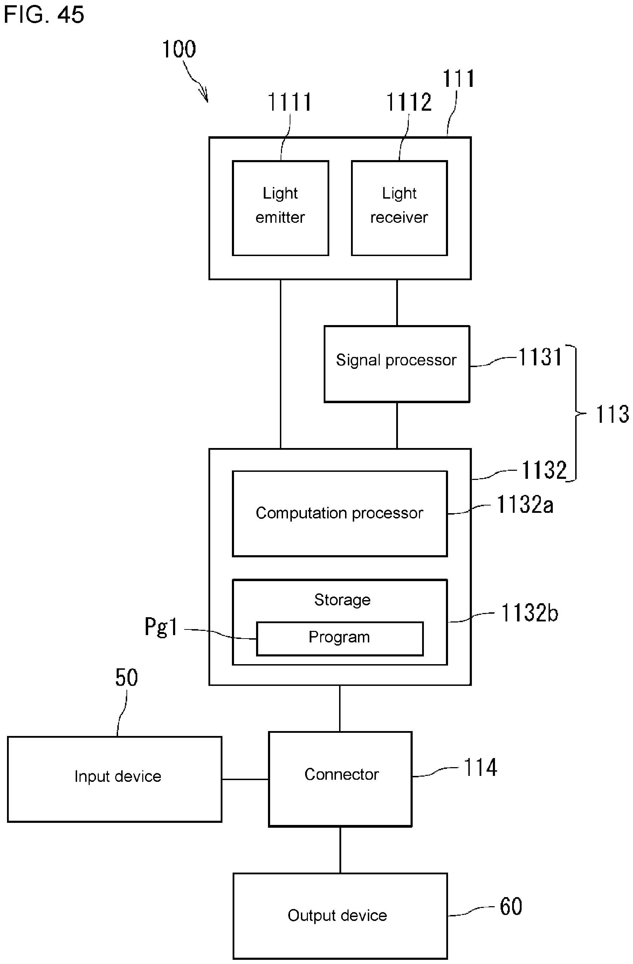

As illustrated in FIG. 45, the measurement device 100 according to each of the above embodiments may include, for example, an input device 50 and an output device 60.

The input device 50 is connectable to, for example, the processing unit 113 through the connector 114. In response to, for example, an operation of a user, the input device 50 can transmit input of various conditions (also referred to as measurement conditions) for measurement of the flow quantitative value with the measurement device 100 to the processing unit 113. Examples of the measurement conditions include the frequency range in the frequency spectrum calculated by the computation processor 1132a. Examples of the input device 50 include an operation portion such as a keyboard, a mouse, a touchscreen, and a switch, and a microphone for voice input. The input device 50 allows a user to easily set intended measurement conditions. This may improve the user convenience of the measurement device 100. The measurement conditions may also include, for example, the light amount or intensity of irradiation light L1 emitted from the light emitter 1111, the period in which the light receiver 1112 outputs a signal, the sampling rate in AD conversion, and an operation expression for calibration data. The input device 50 may also allow input of various items of information about the fluid 3 such as the viscosity, concentration, or the size of a scatterer in the fluid 3.

The output device 60 is connectable to, for example, the processing unit 113 through the connector 114. The output device 60 may include, for example, a display that visibly outputs various items of information about measurements of the flow quantitative value or a speaker that audibly outputs various items of information about measurements of the flow quantitative value. Examples of the display include a liquid crystal display and a touchscreen. When the input device 50 includes a touchscreen, the displays of the input device 50 and the output device 60 may be a single touchscreen. The measurement device 100 with this structure includes fewer components, has a smaller size, and facilitates manufacture. The display may visibly display the measurement conditions, the frequency spectrum, or the mean frequency fm or flow quantitative value as the measurement results. This allows a user to easily view the various items of information about measurements of the flow quantitative value. For example, the display may allow a user to change the output form of various items of information in the output device 60 through the input device 50. The change in the output form may include, for example, a change in the display form and switching of displayed information. The display thus allows a user to easily view the various items of information about measurements of the flow quantitative value. This may improve the user convenience of the measurement device 100. 2-11. Twelfth Embodiment

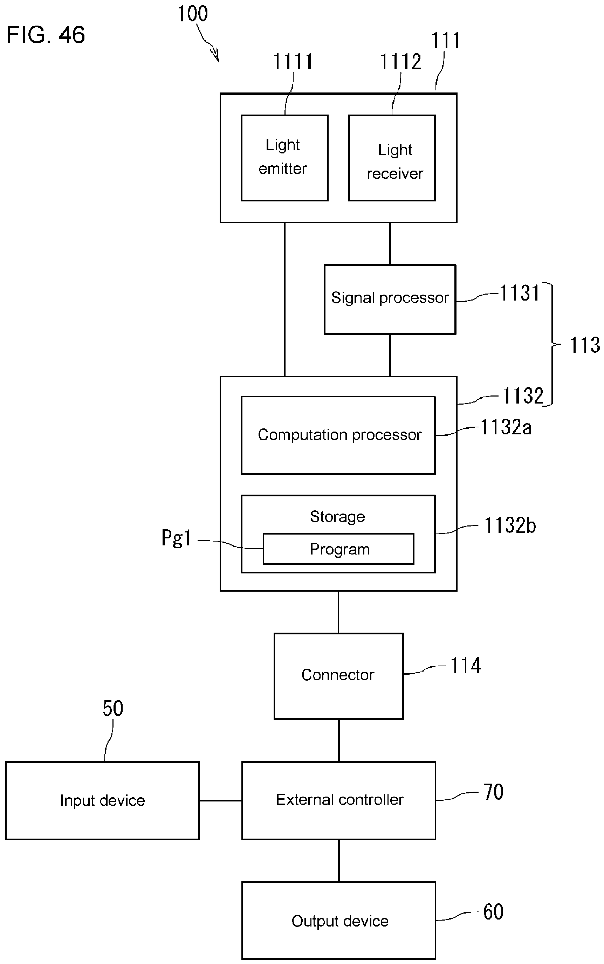

As illustrated in FIG. 46, the measurement device 100 according to each of the above embodiments may further include, for example, an external controller 70. The external controller 70 may include, for example, a computer such as a microcomputer.

The external controller 70 stores measurement conditions such as the light amount or intensity of irradiation light L1, the period in which the light receiver 1112 outputs a signal, and the sampling rate in AD conversion. These measurement conditions may be input into the processing unit 113. This reduces, for example, the processes to be performed by the computation processor 1132a, thus improving the processing speed of the processing unit 113. The measurement conditions include, for example, the same conditions as the various conditions for the measurement of the flow quantitative value with the measurement device 100 that can be input by the input device 50.

The external controller 70 may control, for example, the input device 50 and the output device 60. This structure reduces the number of units having various functions (also referred to as functional units) controlled by the processing unit 113, thus improving the processing speed of the processing unit 113. The external controller 70 may include, for example, various other functional units including multiple electronic components. Examples of the various other functional units include a pressure gauge and a thermometer. This may improve, for example, the design flexibility and the user convenience of the measurement device 100.

The external controller 70, the processing unit 113, the input device 50, and the output device 60 may communicate with one another with wires or wirelessly. The processing unit 113 and the external controller 70 communicate with each other in accordance with, for example, any telecommunications standards. Such telecommunications standards include, for example, Inter-Integrated Circuit (IIC), the Serial Peripheral Interface (SPI), and a universal asynchronous receiver transmitter (UART).

The sensor 111, the signal processor 1131, and the external controller 70 may directly communicate with one another. In this case, the measurement device 100 may eliminate the processing unit 113, and the external controller 70 may serve as the processing unit 113. For example, the sensor 111 and the external controller 70 may communicate directly with each other to eliminate delays in signal transmission between the processing unit 113 and the external controller 70. The measurement device 100 can thus have, for example, higher processing speed. This may improve the user convenience of the measurement device 100. 2-12. Thirteenth Embodiment

In each of the above embodiments, the measurement device 100 may be replaced with a measurement system 200 that includes at least two functional components of the measurement device 100 connected to communicate with each other. As illustrated in FIG. 47, the measurement system 200 in a thirteenth embodiment includes, for example, the light emitter 1111, the light receiver 1112, the signal processor 1131, and the information processor 1132 including the computation processor 1132a. In the example of FIG. 47, the light emitter 1111 and the light receiver 1112, the light emitter 1111 and the information processor 1132, the light receiver 1112 and the signal processor 1131, and the signal processor 1131 and the information processor 1132 are connected to allow communication between them.

3. Others

In each of the above second, fourth, fifth, and seventh embodiments, the reflector 121 may be located, for example, within the flow path 22.

In each of the above first, third, sixth, eighth, and tenth embodiments, the reflector 121 and the second housing 122 included in the second section 12 may be, for example, separate members, rather than an integral piece. In this case, the reflector 121 may be located along, for example, the outer surface of the tubular body serving as the flow path section 21, and the first surface 112f of the first housing 112 and the second surface 122f of the second housing 122 may be located adjacent to or in contact with each other. This allows the sensor 111 and the reflector 121 to face each other across the flow path 22 as in the first state. The reflector 121 may be, for example, attached or bonded along the outer surface of the tubular body serving as the flow path section 21.

In each of the above embodiments, the calibration data may indicate, for example, the relationship between a value obtained by processing the mean frequency fm with a predetermined arithmetic operation (also referred to as a flow calculation value) and a quantitative value for the flow of the fluid 3 (flow quantitative value). In this case, the computation processor 1132a can calculate, for example, the quantitative value for the flow of the fluid 3 (flow quantitative value) based on the flow calculation value obtained by processing the mean frequency fm with the predetermined arithmetic operation and prepared calibration data.

In each of the above embodiments, the flow path section 21 may include a tubular body different from a pipe in any devices, and may include, for example, a blood vessel in a living body.

In each of the above embodiments, at least one of the functions of the computation processor 1132a may be implemented with hardware such as a dedicated electronic circuit.

The components described in the above embodiments and variations may be entirely or partially combined as appropriate unless any contradiction arises.

REFERENCE SIGNS

1 measurement module
3 fluid
15 optical filter
16 cover
21 flow path section
21i internal space
21t light transmitter
22 flow path
100, 900 measurement device
110 housing
110r fourth recess
111 sensor
1111 light emitter
1112 light receiver
Ills light shield

112 first housing
112f first surface
112r third recess
121 reflector
122 second housing
122f second surface
Gp1gap
L1 irradiation light
L2 coherent light
Op1first opening
Op2second opening
Op3third opening

**Claims**

1. A measurement module for measuring a fluid on a flow path defined in an internal space of a flow path section, the measurement module comprising:

   a sensor including a light emitter and a light receiver; and
   a reflector,
   wherein the sensor and the reflector are placeable into a first state in which the sensor and the reflector face each other across the flow path, and
   in the first state, the light receiver receives, in response to the light emitter irradiating the flow path section with light, light scattered by the flow path section, light transmitted through the flow path section and scattered by the fluid on the flow path, and light transmitted through the flow path section and the fluid on the flow path and reflected by the reflector.

2. The measurement module according to claim 1, further comprising:

   an optical filter,
   wherein the optical filter allows transmission of light in a first wavelength range including a wavelength of the light from the light emitter and allows transmission of less light in a second wavelength range different from the first wavelength range, and the optical filter is between the flow path section and the light receiver in the first state.

3. The measurement module according to claim 1 or claim 2, further comprising:

   a first housing and a second housing each having higher light-shielding than the flow path section,
   wherein the first housing and the second housing are placeable into a third state in which the first housing and the second housing are located across the flow path section in the first state,
   the first housing includes a recess to surround the sensor from a position opposite to the flow path section in the third state, and a first surface adjacent to an opening of the recess and placeable along the second housing in the third state, and
   the second housing includes a second surface placeable along the first surface in the third state.

4. The measurement module according to claim 3, wherein
   the first surface and the second surface each are bent in a direction away from an area in which the flow path section is located in the third state.

5. The measurement module according to claim 3 or claim 4, further comprising:

   a cover,
   wherein the cover covers a gap between the first surface and the second surface in the third state.

6. The measurement module according to any one of claims 3 to 5, wherein

   the first housing and the second housing together include, in the third state, a first opening at a first end of the flow path section in a longitudinal direction of the flow path section and a second opening at a second end of the flow path section opposite to the first end in the longitudinal direction, and the flow path section extends through the first opening and the second opening,

the sensor includes a light shield, and

the light shield obstructs, in the third state, an optical path of light traveling from outside the measurement module through at least one of the first opening or the second opening toward the light receiver.

7. A measurement module, comprising:

a flow path section including, in an internal space of the flow path section, a flow path on which a fluid flows;

a sensor including a light emitter and a light receiver; and

a reflector facing the sensor across at least a part of the flow path,

wherein the light emitter irradiates the flow path section with light,

the flow path section includes a light transmitter to transmit the light from the light emitter, and

the light receiver receives light emitted from the light emitter and scattered by the flow path section, light emitted from the light emitter, transmitted through the light transmitter, and scattered by the fluid on the flow path, and light emitted from the light emitter, transmitted through the light transmitter and the fluid on the flow path, and reflected by the reflector.

8. The measurement module according to claim 7, wherein

the reflector is located on a portion of an outer surface of the flow path section opposite to the flow path or outside the flow path section.

9. The measurement module according to claim 7 or claim 8, further comprising:

an optical filter between the flow path and the light receiver,

wherein the optical filter allows transmission of light in a first wavelength range including a wavelength of the light from the light emitter and allows transmission of less light in a second wavelength range different from the first wavelength range.

10. The measurement module according to any one of claims 7 to 9, further comprising:

a housing including at least a part of the flow path section and surrounding the flow path, or surrounding the flow path section to obstruct an optical path of light traveling from outside the measurement module toward the light receiver.

11. The measurement module according to claim 10, wherein

the housing includes a first opening at a first end of the flow path in a longitudinal direction of the flow path and a second opening at a second end of the flow path opposite to the first end in the longitudinal direction, and

the sensor includes a light shield configured to obstruct an optical path of light traveling from outside the measurement module through at least one of the first opening or the second opening toward the light receiver.

12. A measurement device, comprising:

the measurement module according to any one of claims 1 to 11; and

a processing unit,

wherein the light receiver receives light and outputs a signal corresponding to an intensity of the light, and

the processing unit calculates a quantitative calculation value for a flow state of the fluid based on the signal output from the light receiver.

FIG. 1

FIG. 2

FIG. 3

FIG. 4

# FIG. 5
## V–V

FIG. 6

## FIG. 7

FIG. 8

FIG. 9

FIG. 10

FIG. 11

FIG. 12

FIG. 13

FIG. 14

XIV-XIV

FIG. 15

FIG. 16

FIG. 17

FIG. 18

FIG. 19

FIG. 21

FIG. 22

FIG. 23

FIG. 24

FIG. 25

FIG. 26

FIG. 27

FIG. 28

FIG. 29

# FIG. 30

XXX-XXX

## FIG. 31

FIG. 32

FIG. 33

FIG. 34

FIG. 35

FIG. 36

FIG. 37

FIG. 38

FIG. 39

FIG. 40

FIG. 41

FIG. 42

FIG. 43

FIG. 44

FIG. 45

FIG. 46

FIG. 47

200

1111

1112    111

Light emitter

Light receiver

Signal processor — 1131

Computation processor — 1132, 1132a

Storage — 1132b

Pg1 — Program

FIG. 48

Flow rate calculation value

Flow rate set value

| | INTERNATIONAL SEARCH REPORT | International application No. |
|---|---|---|
| | | PCT/JP2021/024345 |

**A. CLASSIFICATION OF SUBJECT MATTER**
Int. Cl. G01F1/66(2006.01)i, A61B5/026(2006.01)i
FI: G01F1/66 103, A61B5/026 120

According to International Patent Classification (IPC) or to both national classification and IPC

**B. FIELDS SEARCHED**

Minimum documentation searched (classification system followed by classification symbols)
Int. Cl. G01F1/66, G01F1/00, G01P5/00, G01P5/26, A61B5/026

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2014/136242 A1 (PIONEER CORP.) 12 September 2014 (2014-09-12), paragraphs [0055]-[0064], fig. 4, 5 | 1-12 |
| A | WO 2016/132512 A1 (AICHI TOKEI DENKI CO., LTD.) 25 August 2016 (2016-08-25), paragraphs [0052]-[0061], fig. 8-10 | 1-12 |

☐ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09.07.2021 | 03.08.2021 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

Information on patent family members

International application No.

PCT/JP2021/024345

| Patent Documents referred to in the Report | Publication Date | Patent Family | Publication Date |
|---|---|---|---|
| WO 2014/136242 A1 | 12.09.2014 | JP 2018-126520 A<br>JP 2020-072823 A | |
| WO 2016/132512 A1 | 25.08.2016 | JP 6404446 B2 | |

Form PCT/ISA/210 (patent family annex) (January 2015)

**REFERENCES CITED IN THE DESCRIPTION**

**Patent documents cited in the description**

• WO 2013153664 A